# EUROPEAN PATENT APPLICATION

(11) **EP 4 385 448 A1**
(43) Date of publication of application: **19.06.2024**
(21) Application number: 22869266.1
(22) Date of filing: 14.09.2022
(51) Int. Cl.: A61B 34/20

(54) **METHOD AND SYSTEM FOR CALCULATING SPATIAL REGISTRATION POSE OF SURGICAL ROBOT**

(30) Priority: 14.09.2021 CN 202111072944; 21.04.2022 CN 202210423164
(71) Applicant: Wuhan United Imaging Healthcare Surgical Technology Co., Ltd., Wuhan, Hubei 430073 (CN)
(72) Inventor: ZHANG, Zhesi, Wuhan, Hubei 430073 (CN); ZHANG, Jin, Wuhan, Hubei 430073 (CN)
(74) Representative: Wang, Bo
(86) International application number: PCT/CN2022/118745
(87) International publication number: WO 2023/040897

(57) **Abstract**

A method (200) and system for calculating a spatial registration pose of a surgical robot. The method (200) comprises: determining a target field-of-view range of a navigator (210), adjusting the pose of the navigator on the basis of the target field-of-view range (220); and determining a spatial registration pose of a surgical robot according to the target field-of-view range and the pose of an end instrument of a robot arm of the surgical robot (230).

## Description

### CROSS-REFERENCE TO RELATED DISCLOSURES

This application claims priority to Chinese Patent Disclosure No. 202111072944.6, filed on September 14, 2021, and priority to Chinese Patent Disclosure No. 202210423164.X, filed on April 21, 2022, the contents of each of which are incorporated herein by reference in their entirety.

### TECHNICAL FIELD

The present disclosure relates to the field of medical devices, and in particular to methods and systems for determining space registration poses of surgical robots.

### BACKGROUND

Surgical precision is crucial for surgery. To meet clinical needs, various types of surgical robots have emerged, and the surgical robots can significantly improve the surgical precision relative to conventional surgery. However, before a navigation device that uses the surgical robot performs real-time detection and tracking of a target object, it is usually necessary for an operator to adjust the pose of the navigation device to a desired state according to experience, thereby increasing the workload of medical staff and the cost of surgery, and the efficiency is relatively low.

Accordingly, it is desirable to provide a method and system for determining a space registration pose of a surgical robot for quickly and accurately determining a space registration pose of the surgical robot.

### SUMMARY

According to one of the embodiments of the present disclosure, a method for determining a space registration pose of a surgical robot is provided, the method include: determining a target field of view of a navigation device, adjusting a pose of the navigation device based on the target field of view, and determining the space registration pose of the surgical robot according to the target field of view and a pose of an end effector of the mechanical arm of the surgical robot.

In some embodiments, by determining a target field of view of the navigation device, adjusting a pose of the navigation device based on the target field of view, and determining the space registration pose of the surgical robot according to the target field of view and a pose of the end effector of the mechanical arm of the surgical robot, the end effector of the mechanical arm of the surgical robot is prevented from being located outside the target field of view of the navigation device and resulting in a registration failure, at the same time, making the end effector of the mechanical arm of the surgical robot be located within a relatively great field of view (i.e., the target field of view) of the navigation device to improve a registration accuracy.

In some embodiments, the determining the target field of view of the navigation device include: obtaining an initial receptive field of the navigation device, obtaining a space range constraint, and obtaining the target field of view of the navigation device by constraining the initial receptive field according to the space range constraint.

In some embodiments, by obtaining the initial receptive field of the navigation device and the space range constraint and constraining the initial receptive field according to the space range constraint, the target field of view of the navigation device is quickly and accurately determined.

In some embodiments, the space range constraint includes at least one of a constraint of the mechanical arm, a constraint of a target object, and a constraint of a field of view of the navigation device.

In some embodiments, the obtaining the space range constraint, and obtaining the target field of view of the navigation device by constraining the initial receptive field according to the space range constraint include: constructing an objective function for describing a space range of the target field of view; performing a constraint calculation on the objective function according to the space range constraint through an optimization algorithm; and determining the target field of view based on the constrained objective function.

In some embodiments, by constructing the objective function for describing the space range of the target field of view and performing the constraint calculation on the objective function according to the space range constraint through the optimization algorithm, a relatively accurate target field of view is quickly determined based on the constrained objective function.

In some embodiments, the adjusting the pose of the navigation device based on the target field of view includes: after determining the target field of view of the navigation device, adjusting a positioning of the navigation device according to the target field of view to make the end effector of the mechanical arm of the surgical robot be located within the target field of view.

In some embodiments, after the target field of view of the navigation device is determined, the positioning of the navigation device is adjusted according to the target field of view to make the end effector of the mechanical arm of the surgical robot be located within the target field of view to cause the end effector of the mechanical arm of the surgical robot to always be located within the target field of view when the space registration of the surgical robot is performed in a subsequent time, avoiding a registration failure.

In some embodiments, the determining the space registration pose of the surgical robot based on the target field of view and the pose of the end effector of the mechanical arm of the surgical robot includes: obtaining a plurality of position coordinates and a plurality of rotation postures of the end effector of the mechanical arm; and determining the space registration pose of the surgical robot based on the plurality of position coordinates and the plurality of rotation postures.

In some embodiments, the plurality of initial rotation postures are deflected to a same direction by obtaining a plurality of initial rotation postures of the end effector of the mechanical arm to obtain the plurality of rotation postures quickly.

In some embodiments, the obtaining the plurality of rotation postures of the end effector of the mechanical arm includes: obtaining the plurality of initial rotation postures of the end effector of the mechanical arm; and obtaining the plurality of rotation postures by deflecting the plurality of initial rotation postures to the same direction.

In some embodiments, the obtaining the plurality of initial rotation postures of the end effector of the mechanical arm include: obtaining a maximum posture range of the end effector of the mechanical arm; and determining the plurality of initial rotation postures of the end effector of the mechanical arm in the maximum posture range according to a count of postures and/or a posture dispersion of the end effector of the mechanical arm.

In some embodiments, the method further include: obtaining a transformation relationship between various systems of the surgical robot.

In some embodiments, the adjusting a pose of the navigation device based on the target field of view include: before determining the space registration pose of the surgical robot, obtaining a position relationship between at least one of one or more reference markers of the surgical robot and the target field of view of the navigation device; and generating pose adjustment data based on the position relationship and adjusting the pose of the navigation device based on the pose adjustment data to make the at least one of the one or more reference markers fall within the target field of view of the navigation device.

In some embodiments, by obtaining a position relationship between the at least one of one or more reference markers of the surgical robot and the target field of view of the navigation device, the pose adjustment data is generated more quickly and accurately based on the position relationship, and the pose of the navigation device is adjusted based on the pose adjustment data to reduce the risk of a failure of space registration pose caused by a poor field of view of the navigation device.

In some embodiments, the generating the pose adjustment data based on the position relationship and adjusting the pose of the navigation device based on the pose adjustment data includes: determining a relative position relationship map and the pose adjustment information between an identifier corresponding to at least one of the one or more reference markers and the identifier corresponding to the target field of view based on the position relationship, and adjusting the pose of the navigation device according to the relative position relationship map and the pose adjustment information.

In some embodiments, the pose adjustment data generated by the position relationship automatically adjusts the pose of the navigation device directly without relying on manual experience to adjust the pose of the navigation device, which not only improves the accuracy of adjusting the pose of the navigation device, but also improves the efficiency of adjusting the pose of the navigation device, and at least one of the one or more reference markers of the surgical robot is located within the target field of view of the navigation device after adjustment, which ensures that the at least one of the one or more reference markers of the surgical robot is always located within the target field of view during subsequent space registration processes to avoid the registration failure. In some embodiments, the adjusting the pose of the navigation device according to the relative position relationship map and the pose adjustment information includes: adjusting the pose of the navigation device based on the pose adjustment information and obtaining a new position relationship between the at least one of the one or more reference markers and the target field of view of the navigation device; determining a current relative position relationship map between the identifier corresponding to the at least one of the one or more reference markers and the identifier corresponding to the target field of view based on the new position relationship; and in response to determining that the at least one of the one or more reference markers is not located within the target field of view determined based on the current relative position relationship map, adjusting the pose of the navigation device based on the current relative position relationship map to make the at least one of the one or more reference markers fall within the target field of view of the navigation device.

In some embodiments, by adjusting the pose of the navigation device based on the pose adjustment information and obtaining the new position relationship between the at least one of the one or more reference markers and the target field of view of the navigation device; determining a current relative position relationship map between the identifier corresponding to the at least one of the one or more reference markers and the identifier corresponding to the target field of view based on the new position relationship; and in response to determining that the at least one of the one or more reference markers is not located within the target field of view determined based on the current relative position relationship map, adjusting the pose of the navigation device based on the current relative position relationship map, the position relationship between the at least one of the one or more reference markers of the surgical robot and the target field of view of the navigation device is obtained more intuitively after each adjustment of the pose of the navigation device, thereby ensuring that the at least one of the one or more reference markers of the surgical robot is located within the target field of view after the adjustment of the pose of the navigation device.

In some embodiments, and the generating pose adjustment data based on the position relationship and the adjusting the pose of the navigation device based on the pose adjustment data includes: adjusting the pose of the navigation device based on the pose adjustment data to make the first reference marker fall within a first visual field region and make the second reference marker fall within a second visual field region; wherein the first reference marker and the second reference marker are respectively disposed on different portions of the surgical robot, the second visual field region is the target field of view or located within the target field of view, and the first visual field region is located within the target field of view.

In some embodiments, the one or more reference markers includes the first reference marker and the second reference marker, generating the pose adjustment data based on the position relationship and adjusting the pose of the navigation device based on the pose adjustment data includes: adjusting the pose of the navigation device based on the pose adjustment data to make the first reference marker fall within the first visual field region; and adjusting the pose of the end effector of the mechanical arm based on the pose adjustment data to make the second reference marker disposed at an end of the mechanical arm fall within the second visual field region; wherein the first reference marker is disposed on a surgical trolley of the surgical robot or the target object, the second reference marker is disposed at the end of the mechanical arm, the second visual field region is the target field of view, and the first visual field region is located within the second visual field region.

In some embodiments, the pose of the navigation device is adjusted based on the pose adjustment data to make the first reference markers fall within the first visual field region, so that the first reference marker is always located within the target field of view during subsequent space registration ranges; after a pose adjustment of the navigation device is completed, the pose of the end effector of the mechanical arm is adjusted based on the pose adjustment data, which makes the second reference marker disposed at an end of the mechanical arm fall within a second visual field region more quickly and accurately, ensuring a smooth completion of the subsequent space registration, and avoiding the risk of the space registration failure caused by the poor visual field of the navigation device.

In some embodiments, the method further includes: displaying the relative position relationship map in a first display region, and displaying a target pose in a second display region.

In some embodiments, the method further includes: obtaining pose information of the navigation device through a pose monitoring device; and generating adjustment feedback information based on the pose information of the navigation device, wherein the adjustment feedback information includes moving distance information of the navigation device and moving velocity information of the navigation device; and displaying the adjustment feedback information on a display interface.

In some embodiments, the adjustment feedback information is displayed on the display interface, which provides a reference for a user (e.g., a doctor, a nurse, etc.) to further determine an adjustment effect of the pose of the navigation device.

In some embodiments, the obtaining the position relationship between the at least one of the one or more reference markers of the surgical robot and the target field of view of the navigation device includes: obtaining position information of the at least one of the one or more reference markers of the surgical robot relative to the navigation device; obtaining the target field of view of the navigation device; and determining the position relationship between the at least one of the one or more reference markers and the target field of view based on the position information.

According to one embodiment of the present disclosure, a system for determining a space registration pose of a surgical robot is provided, the system includes: a range determination module configured to determine a target field of view of a navigation device; a pose determination module configured to adjust a pose of the navigation device based on the target field of view; and the pose registration module configured to determine a space registration pose of a surgical robot according to the target field of view, a target pose of the navigation device, and an initial pose of an end effector of a mechanical arm of the surgical robot.

According to one of the embodiments of the present disclosure, a device for determining a space registration pose of a surgical robot is provided, the device includes a processor, and the processor is configured to perform the above method for determining the space registration pose of the surgical robot.

According to one of the embodiments of the present disclosure, a non-transitory computer-readable storage medium is provided, the storage medium stores computer instructions, and the computer executing the above method for determining a space registration pose of the surgical robot when the computer reads the computer instructions in the storage medium.

According to one of the embodiments of the present disclosure, a method for obtaining a visual navigation device receptive field is provided, the method includes: obtaining a first receptive field of a navigation device in the surgical robot; obtaining a space range constraint, and obtaining a second receptive field of the navigation device by constraining the first receptive field according to the space range constraint.

In some embodiments, the space range constraint includes at least one of a constraint of the mechanical arm, a constraint of a target object, and a constraint of a field of view of the navigation device.

In some embodiments, the obtaining a second receptive field of the navigation device by constraining the first receptive field according to the space range constraint includes: constructing an objective function for describing a space range of the second receptive field; performing a constraint calculation on the objective function according to the space range constraint through an optimization algorithm; and determining the second receptive field according to the constrained objective function.

In some embodiments, after the second receptive field of the navigation device is obtained, the method includes: adjusting the positioning of the navigation device according to the second receptive field to make the end effector of the mechanical arm of the surgical robot located within the second receptive field.

According to one embodiment of the present disclosure, a method for determining a space registration pose of a surgical robot is provided, the method includes: obtaining a first receptive field of a navigation device; obtaining a space range constraint, and obtaining a second receptive field of the navigation device by constraining an initial receptive field according to the space range constraint; and determining a space registration pose of the surgical robot according to the second receptive field and a pose of an end effector of a mechanical arm of the surgical robot.

In some embodiments, the determining the space registration pose of the surgical robot according to the second receptive field and the pose of the end effector of the mechanical arm of the surgical robot includes: obtaining a plurality of position coordinates and a plurality of rotation postures of the end effector of the mechanical arm, and the plurality of rotation postures form a target direction set; and determining the space registration pose of the surgical robot based on the plurality of position coordinates and the plurality of rotation postures in the target direction set, wherein the plurality of position coordinates correspond to the plurality of rotation postures, respectively.

In some embodiments, the obtaining the plurality of position coordinates and the plurality of rotation postures of the end effector of the mechanical arm, and the plurality of rotation postures form the target direction set includes: obtaining an initial direction set of the end effector of the mechanical arm, the initial direction set includes a plurality of initial rotation postures; and obtaining the target direction set of the end effector of the mechanical arm.

In some embodiments, the obtaining an initial direction set of the end effector of the mechanical arm includes: obtaining a maximum posture range of the end effector of the mechanical arm identified by the navigation device; and determining the plurality of initial rotation postures of the end effector of the mechanical arm in the maximum posture range according to the count of postures and/or the posture dispersion of the end effector of the mechanical arm.

In some embodiments, before obtaining the space range constraint and obtaining the second receptive field of the navigation device by constraining the first receptive field according to the space range constraint, the method further includes: obtaining a transformation relationship between various coordinate systems in the surgical robot, wherein the various coordinate systems of the surgical robot includes at least a portion of coordinate systems of the following coordinate systems: a mechanical arm coordinate system, a navigation device coordinate system, an coordinate system of the end of the mechanical arm, and a coordinate system of an end sensor of the mechanical arm.

According to one embodiment of the present disclosure, a surgical robot is provided, the surgical robot includes a navigation device, a mechanical arm, and a processor, the processor obtains a first receptive field of the navigation device; the processor obtains a space range constraint and obtain a second receptive field of the navigation device by constraining the first receptive field according to the space range constraint.

According to one of the embodiments of the present disclosure, a device for obtaining a visual navigation device receptive field is provided, the device includes an obtaining module and a constraint module, the obtaining module is configured to obtain a first receptive field of a navigation device in a surgical robot, and the constraint module is configured to obtain a space range constraint, and obtain a second receptive field of the navigation device by constraining the first receptive field according to the space range constraint.

According to one of the embodiments of the present disclosure, an electronic device is provided, including a memory and a processor, the memory stores computer programs, and the processor is set to execute the method for obtaining a visual navigation device receptive field or the method for determining a space registration pose of the surgical robot.

According to one of the embodiments of the present disclosure, a storage medium is provided, the storage medium stores the computer programs, and the computer programs are set to execute, when operating, the operations of the method for obtaining a visual navigation device receptive field or the method for determining a space registration pose of the surgical robot.

According to one of the embodiments of the present disclosure, a method for adjusting a pose of an optical navigation device is provided, the method includes: obtaining a position relationship between the at least one of the one or more reference markers of the surgical robot and the target visual field region of the optical navigation device of the surgical robot; generating the pose adjustment data based on the position relationship, and adjusting a pose of the optical navigation device based on the pose adjustment data to make the reference marker fall within the target visual field region of the optical navigation device.

In some embodiments, the generating the pose adjustment data based on the position relationship, and adjusting the pose of the optical navigation device based on the pose adjustment data to make the reference marker fall within the target visual field region of the optical navigation device includes: determining the relative position relationship map between the identifier corresponding to the at least one of the one or more reference markers and the identifier corresponding to the target visual field region according to the position relationship; and adjusting the pose of the optical navigation device based on the relative position relationship map to make the at least one of the one or more reference markers fall within the target visual field region of the optical navigation device.

In some embodiments, the generating the pose adjustment data based on the position relationship, and adjusting the pose of the optical navigation device based on the pose adjustment data to make the reference marker fall within the target visual field region of the optical navigation device further includes: determining the pose adjustment information of the optical navigation device based on the position relationship; adjusting the pose of the optical navigation device based on the pose adjustment information to make the at least one of the one or more reference markers to fall within the target visual field region of the optical navigation device.

In some embodiments, the generating the pose adjustment data based on the position relationship, and adjusting the pose of the optical navigation device based on the pose adjustment data to make the reference marker fall within the target visual field region of the optical navigation device further includes: determining, based on the position relationship, a relative position relationship map between the identifier corresponding to the at least one of the one or more reference markers and the identifier corresponding to the target visual field region and the pose adjustment information of the optical navigation device; and adjusting the pose of the optical navigation device based on the pose adjustment information to make the at least one of the one or more reference markers to fall within the target visual field region of the optical navigation device.

In some embodiments, the adjusting the pose of the optical navigation device based on the pose adjustment information to make the at least one of the one or more reference markers to fall within the target visual field region of the optical navigation device includes: adjusting the pose of the optical navigation device based on the pose adjustment information and obtaining the new position relationship between the at least one of the one or more reference markers and the target visual field region r of the optical navigation device; determining the current relative position relationship map between the identifier corresponding to the at least one of the one or more reference markers and the identifier corresponding to the target visual field region based on the new position relationship; and in response to determining that the at least one of the one or more reference markers is not located within the target visual field region, adjusting the pose of the optical navigation device based on the current relative position relationship map to make the at least one of the one or more reference markers fall within the target visual field region of the optical navigation device.

In some embodiments, the pose adjustment information includes direction adjustment information and distance adjustment information; the determining the pose adjustment information of the optical navigation device based on the position relationship includes: determining the direction adjustment information of the optical navigation device and the distance adjustment information of the optical navigation device based on the position relationship.

In some embodiments, the adjusting the pose of the optical navigation device based on the pose adjustment information to make the at least one of the one or more reference markers fall within the target visual field region of the optical navigation device includes: adjusting the pose of the optical navigation device based on the pose adjustment data to make first reference marker fall within the first visual field region and the second reference marker fall within a second visual field region; wherein the first reference marker is disposed on the surgical trolley of the surgical robot or an object to be detected of a system for navigation and positioning of the surgical robot, and the second reference marker is disposed at an end of the mechanical arm of the system for navigation and positioning of the surgical robot, and the first visual field region is located within the second visual field region.

In some embodiments, the adjusting the pose of the optical navigation device based on the pose adjustment information to make the at least one of the one or more reference markers to fall within the target visual field region of the optical navigation device includes: adjusting the pose of the optical navigation device based on the pose adjustment data to make the first reference marker fall within the first visual field region; adjusting the pose of the end of the mechanical arm based on the pose adjustment data to make the second reference marker disposed at the end of the mechanical arm fall within the first visual field region; wherein the first reference marker is disposed on the surgical trolley of the surgical robot or the object to be detected of the system for navigation and positioning of the surgical robot, the second reference marker is disposed at the end of the mechanical arm of the system for navigation and positioning of the surgical robot, and the first visual field region is disposed within the second visual field region.

In some embodiments, the method further includes: displaying the relative position relationship map in the first display region, and displaying the pose adjustment information in the second display region.

In some embodiments, the method further includes: obtaining the pose information of the one or more reference markers by the pose monitoring device disposed on the optical navigation device; generating the adjustment feedback information based on the pose information of the one or more reference markers; the adjustment feedback information includes the moving distance information of the one or more reference markers and the moving velocity information of the one or more reference markers; and displaying the adjustment feedback information on the display interface.

In some embodiments, obtaining a position relationship between the at least one of the one or more reference markers of the system for navigation and positioning of the surgical robot and the target visual field region of the optical navigation device of the system for navigation and positioning of the surgical robot includes: obtaining the position information of the at least one of the one or more reference markers of the system for navigation and positioning of the surgical robot relative to the optical navigation device of the system for navigation and positioning of the surgical robot; and obtaining the target visual field region of the optical navigation device; determining the position relationship between the at least one of the one or more reference markers and the target visual field region of the optical navigation device.

According to one of the embodiments of the present disclosure, a system for adjusting a pose of a surgical marker is provided, the system includes one or more reference markers, an optical navigation device, and a computer device, an optical navigation device communicating with the computer device, wherein: the optical navigation device is configured to acquire position information of the reference marker and send the position information to the computer device; and the computer device is configured to determine a position relationship between the at least one of the one or more reference markers and the target visual field region of the optical navigation device; generate the pose adjustment data based on the position relationship, and adjust the pose of the optical navigation device based on the pose adjustment data to make the at least one of the one or more reference markers fall within the target visual field region of the navigation device.

In some embodiments, the one or more reference markers includes a first reference marker and a second reference marker, the first reference marker is disposed on a surgical trolley of the surgical robot or a target object of the system for navigation and positioning of the surgical robot, and the second reference marker is disposed at the end of the mechanical arm of the system for navigation and positioning of the surgical robot.

In some embodiments, the system further includes the display configured to display the pose adjustment data; and the pose adjustment data includes at least one of a relative position relationship map and pose adjustment information, the pose adjustment data being determined based on the position relationship.

According to one of the embodiments of the present disclosure, a computer device is provided, the computer device includes a memory and a processor, the memory stores the computer programs, and the processor implements the above method for adjusting the pose of the optical navigation device when executing the computer programs.

According to one of the embodiments of the present disclosure, a non-transitory computer-readable storage medium is provided, the storage medium stores the computer programs, the computer programs implement the above method for adjusting the pose of the optical navigation device when executed by the processor.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating an application scenario of a system for determining a space registration pose of a surgical robot according to some embodiments of the disclosure;
FIG. 2 is a flowchart illustrating an exemplary method for determining a space registration pose of a surgical robot according to some embodiments of the disclosure;
FIG. 3 is a flowchart illustrating an exemplary process for determining an exemplary target field of view of a navigation device according to some embodiments of the disclosure;
FIG. 4 is a flowchart illustrating an exemplary process for obtaining a target field of view of a navigation device by constraining an initial receptive field according to a space range constraint according to some embodiments of the disclosure;
FIG. 5 is a schematic diagram illustrating a receptive field according to some embodiments of the present disclosure;
FIG. 6 is a flowchart illustrating an exemplary process for determining a space registration pose of a surgical robot according to some embodiments of the disclosure;
FIG. 7 is a flowchart illustrating an exemplary process for determining a registration pose of a surgical robot based on a transformation relationship between various coordinate systems in a surgical robot according to some embodiments of the disclosure;
FIG. 8 is a block diagram illustrating an exemplary hardware structure of a terminal for performing a process for obtaining a visual navigation device receptive field according to some embodiments of the disclosure;
FIG. 9 is a block diagram illustrating an exemplary structure of a device for obtaining a visual navigation device receptive field according to some embodiments of the disclosure;
FIG. 10 is a flowchart illustrating an exemplary process for determining a target pose 500 of a navigation device based on a target field of view according to some embodiments of the disclosure;
FIG. 11 is a flowchart illustrating an exemplary process for determining a position relationship between at least one of one or more reference markers and a target field of view based on position information of the at least one of the one or more reference markers of a surgical robot relative to a navigation device according to some embodiments of the present disclosure;
FIG. 12 is a flowchart illustrating an exemplary process for generating pose adjustment information based on a relative position relationship map and adjusting a pose of a navigation device based on the pose adjustment information according to some embodiments of the present disclosure;
FIG. 13 is a flowchart illustrating an exemplary process for adjusting a pose of an optical navigation device based on pose adjustment information according to some embodiments of the present disclosure;
FIG. 14 is a flowchart illustrating an exemplary process for adjusting a pose of a navigation device based on a relative position relationship map and pose adjustment data according to some embodiments of the present disclosure;
FIG. 15 is a flowchart illustrating an exemplary process for adjusting a pose of a navigation device based on a relative position relationship map and pose adjustment information according to some embodiments of the present disclosure;
FIG. 16 is a schematic diagram illustrating at least one of one or more reference markers according to some embodiments of the present disclosure;
FIG. 17 is a schematic diagram illustrating a first visual field region and a second visual field region according to some embodiments of the present disclosure;
FIG. 18 is a flowchart illustrating an exemplary process for adjusting a pose of a navigation device based on pose adjustment data according to some embodiments of the present disclosure;
FIG. 19 is a schematic diagram illustrating a display interface according to some embodiments of the present disclosure;
FIG. 20 is another schematic diagram illustrating a display interface according to some embodiments of the present disclosure;
FIG. 21 is a flowchart illustrating an exemplary process for generating and displaying adjustment feedback information based on pose information of a navigation device according to some embodiments of the present disclosure;
FIG. 22 is a schematic diagram illustrating a fourth display region according to some embodiments of the present disclosure; and
FIG. 23 is a block diagram illustrating a server according to some embodiments of the present disclosure.

### DETAILED DESCRIPTION

In order to illustrate the technical solutions related to the embodiments of the present disclosure, a brief introduction of the drawings referred to in the description of the embodiments is provided below. Obviously, the drawings described below are only some examples or embodiments of the present disclosure. Those skilled in the art, without further creative efforts, may apply the present disclosure to other similar scenarios according to these drawings. Unless apparent from the locale or otherwise stated, like reference numerals represent similar structures or operations throughout the several views of the drawings

It will be understood that the term "system," "device," "unit," and/or "module" used herein are one method to distinguish different components, elements, parts, sections, or assembly of different levels in ascending order. However, the terms may be displaced by another expression if they achieve the same purpose.

As used in the disclosure and the appended claims, the singular forms "a," "an," "a kind of," and/or "the" may include plural forms unless the content clearly indicates otherwise. In general, the terms "comprise," "comprises," and/or "comprising," "include," "includes," and/or "including," merely prompt to include steps and elements that have been clearly identified, and these steps and elements do not constitute an exclusive listing. The methods or devices may also include other steps or elements.

The flowcharts used in the present disclosure illustrate operations that systems implement according to some embodiments in the present disclosure. It is to be expressly understood, the operations of the flowchart may be implemented not in order. Conversely, the operations may be implemented in an inverted order, or simultaneously. Moreover, one or more other operations may be added to the flowcharts. One or more operations may be removed from the flowcharts.

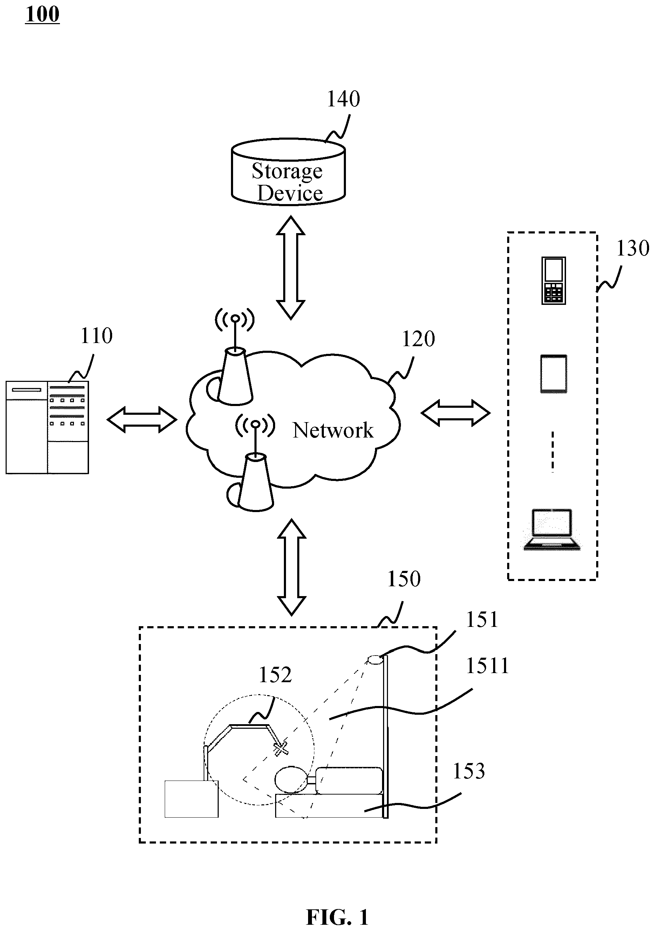

FIG. 1 is a schematic diagram illustrating an application scenario of a system for determining a space registration pose of a surgical robot according to some embodiments of the disclosure.

As shown in FIG. 1, in some embodiments, an application scenario 100 may include a processing device 110, a network 120, a user terminal 130, a storage device 140, and a surgical robot 150. The application scenario 100 may determine a space registration pose of a surgical robot by implementing methods and/or processes disclosed in the present disclosure.

The processing device 110 may be configured to process information and/or data related to the determination of a space registration pose of the surgical robot. For example, the processing device 110 may determine a target field of view of a navigation device 151 of the surgical robot 150, adjust a pose of the navigation device 151 based on the target field of view, determine a space registration pose of the surgical robot 150 based on the target field of view and an end effector of a mechanical arm 152 of the surgical robot 150. As another example, the processing device 110 may obtain an initial receptive field of the navigation device 151 obtain a space range constraint, and obtain a target field of view of the navigation device 151 by constraining the initial receptive field according to the space range constraint. As a further example, the processing device 110 may obtain a position relationship between the end effector of the mechanical arm 152 of the surgical robot 150 and the target field of view of the navigation device 151, generate pose adjustment data according to the position relationship, and control the navigation device 151 to perform a positioning according to the pose adjustment data.

In some embodiments, the processing device 110 may be regional or remote. For example, the processing device 110 may access information and/or materials stored in the user terminal 130, the storage device 140, and the surgical robot 150 through the network 120. In some embodiments, the processing device 110 may be directly connected to the user terminal 130, the storage device 140, and the surgical robot 150 to access the information and/or materials stored therein. In some embodiments, the processing device 110 may include a processor. The processor may process the data and/or information related to the determination of the space registration pose of the surgical robot to perform one or more of the functions described in the present disclosure. For example, the processor may receive a request signal for determining the space registration pose of the surgical robot sent by the user terminal 130 or the surgical robot 150, send a control instruction of controlling the navigation device 151 to perform a positioning to the surgical robot 150.

In some embodiments, the processing device 110 may include a range determination module, a pose determination module, and a pose registration module.

The range determination module may be configured to determine a target field of view of the navigation device. In some embodiments, the range determination module may obtain an initial receptive field of the navigation device 151, obtain a space range constraint, and obtain the target field of view of the navigation device 151 by constraining the initial receptive field according to the space range constraint.

The pose determination module may be configured to adjust the pose of the navigation device 151 based on the target field of view. In some embodiments, the pose determination module may obtain a position relationship between an end effector of the mechanical arm 152 of the surgical robot 150 and the target field of view of the navigation device 151, generate the pose adjustment data based on the position relationship, and adjust the pose of the navigation device 151 based on the pose adjustment data to cause at least one of one or more reference markers to fall within the target field of view of the navigation device 151.

The pose registration module is configured to determine the space registration pose of the surgical robot 150 based on the target field of view, and an initial pose of the end effector of the mechanical arm 152 of the surgical robot 150. In some embodiments, the pose registration module may obtain a plurality of pose pairs of the end effector of the mechanical arm 152, and determine a space registration pose of the surgical robot 150 based on the plurality of pose pairs. Each of the plurality of pose pairs may include a position coordinate and at least one rotation posture corresponding to the position coordinate.

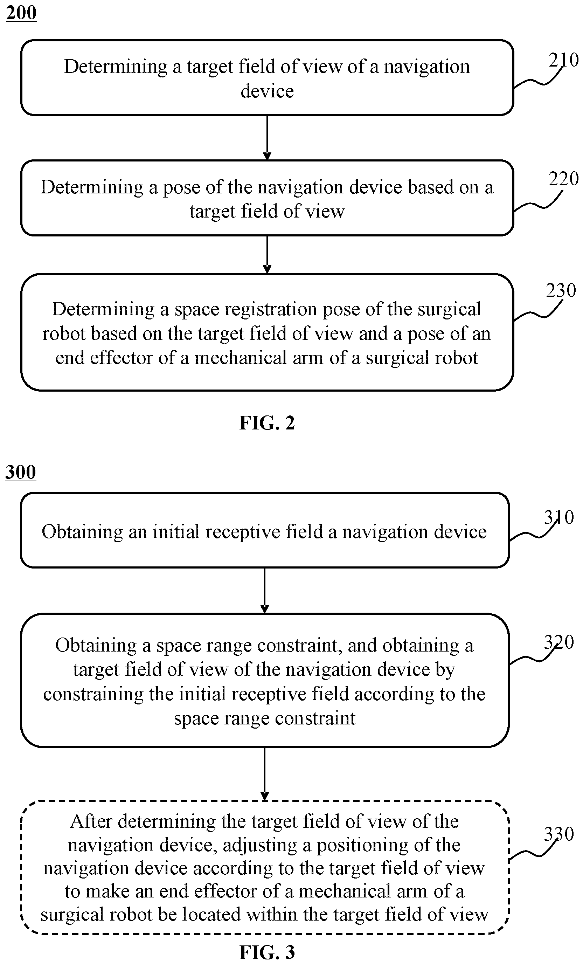

More descriptions of the range determination module, the pose determination module, and the pose registration module may be found in FIG. 2, and the related descriptions, which may not be repeated herein.

It should be noted that the above descriptions of the processing device 110 and its modules are merely provided for the purpose of descriptive convenience and not intended to limit the present disclosure to the scope of the cited embodiments. It should be understood that those skilled in the art, after understanding the principle of the processing device 110, may make any combination of various modules or form a subsystem to be connected to other modules without departing from this principle. In some embodiments, the range determination module, the pose determination module, and the pose registration module disclosed in FIG. 1 may be different modules in a single system, or a single module may implement the functions of two or more of the above modules. For example, various modules may share a storage module, and various modules may respectively have their storage module. Variations such as these are within the scope of protection of the present disclosure.

The network 120 may facilitate the exchange of the data and/or information of the application scenario 100. In some embodiments, one or more components of the application scenario 100 (e.g., the processing device 110, the user terminal 130, the storage device 140, and the surgical robot 150) may send the data and/or information to other components of the application scenario 100 through the network 120. For example, the field of view of the navigation device obtained by the surgical robot 150 may be transmitted to the processing device 110 through the network 120. In some embodiments, the network 120 may be any one or more of a wired network or a wireless network. In some embodiments, the network 120 may include one or more network access points. For example, the network 120 may include one or more wired or wireless network access points (e.g., base stations and/or network switching points), the one or more components of the application scenario 100 may be connected to the network 120 through these network access points to exchange the data and/or information.

The user terminal 130 may input and/or output relevant information or data. A user may obtain the relevant information or data through the user terminal 130. For example, the user may obtain a determined space registration pose of the surgical robot 150 from the processing device 110 through the user terminal 130. In some embodiments, the user terminal 130 may include but is not limited to a smart phone, a tablet computer, a laptop computer, a desktop computer, or the like.

The storage device 140 may store information and/or instructions. In some embodiments, the storage device 140 may store the information and/or instructions obtained from the processing device 110, the user terminal 130, and the surgical robot 150. In some embodiments, the storage device 140 may store the information and/or instructions for execution or use by the processing device 110 to execute the exemplary methods described in the present disclosure. For example, the processing device 110 may process the instructions stored by the storage device 140.

In some embodiments, the storage device 140 may be connected to the network 120 to enable communication with the one or more components of the application scenario 100 (e.g., the processing device 110, the user terminal 130, and the surgical robot 150, etc.). The one or more components of the application scenario 100 may access the information or instructions stored in the storage device 140 through the network 120, determine the target field of view of the navigation device 151 of the surgical robot 150, adjust the pose of the navigation device 151, determine the space registration pose of the surgical robot 150 according to the target field of view of the navigation device 151 of the surgical robot 150. In some embodiments, the storage device 140 may be directly connected or in communication with the one or more components of the application scenario 100 (e.g., the processing device 110, the user terminal 130, and the surgical robot 150, etc.).

The surgical robot 150 may be a device for assisting in performing a surgery, e.g., the surgical robot may assist a doctor in performing a neurosurgical surgery, an orthopedic surgery, or the like. The surgical robot 150 may detect and track a target object (e.g., a site to be operated of the target object, the end effector of the mechanical arm 152, etc.) in real-time during surgery, thereby assisting the surgeon in performing the surgical operation. In some embodiments, the surgical robot 150 includes the navigation device 151, the mechanical arm 152, a support device 153 of the target object (e.g., a scanning bed, a surgical bed, a surgical trolley, etc.), and one or more reference markers (not shown in the figures). The navigation device is configured to obtain a position of the end effector of the mechanical arm 152 of the surgical robot and a position of the site to be operated of the target object on the support device 153 of the target object. In some embodiments, the navigation device 151 may include an optical navigation device, an electromagnetic navigation device, or the like. The optical navigation device includes a binocular optical navigation device, a tri-camera optical navigation device, a structural optical navigation device, or the like.

The mechanical arm 152 may be a device for disposing an instrument, and the instrument may be disposed at the end of a mechanical arm 152. The instrument may be one or more reference markers (e.g., a second reference marker), a surgical instrument, etc. The one or more reference markers at the end of the mechanical arm 152 may be one or more optical markers easy to recognize. For example, an optical blob that reflects near-infrared light. In some embodiments, when the mechanical arm 152 is provided with a surgical instrument at an end of the mechanical arm 152, the one or more reference markers may be provided on the surgical instrument at the end of the mechanical arm 152.

The target object may include a human body, an animal, a molded body, or the like. The site to be operated of the target object on the support device 153 of the target object may include but are not limited to, a head, a limb, an abdomen, or the like, and the target object on the support device 153 of the target object is provided with one or more reference markers. For example, the site to be operated of the target object of the target object may be provided with one or more reference markers.

In some embodiments, before performing an assistance surgery on the site to be operated of the target object on the support device 153 of the target object, the surgical robot 150 needs to perform a space registration on the mechanical arm 152 within the target field of view, one or more markers on the site to be operated of the target object are tracked on the support device 153 of the target object and the end effector of the mechanical arm 152 (e.g., the one or more reference markers) by using the navigation device, which may obtain a real-time pose of the end effector of the mechanical arm 152 and a real-time pose of the site to be operated of the target object to match a position of the end effector of the mechanical arm 152 with a position of the site to be operated of the target object on the support device of the target object, thereby improving the accuracy of the surgery. The one or more reference markers on the end effector of the mechanical arm 152 and the one or more reference markers on the target object need to remain within the field of view of the navigation device 151. In some embodiments, the reference markers on the target object need to be located within an optimal small range (e.g., a first visual field region) of the field of view of the navigation device 151. The one or more reference markers disposed at the end of the mechanical arm 152 are located within the field of view of the navigation device 151, which may not be limited herein. In some embodiments, the one or more reference markers disposed at the end of the mechanical arm 152 need to be located within an optimal small range of the field of view of the navigation device 151. For example, the one or more reference markers disposed at the end of the mechanical arm 152 need to be located within an optimal small range of the field of view of the navigation device 151 that is determined based on the space range constraint. Positions of the one or more reference markers on the target object within the field of view of the navigation device 151 may not be limited.

In some embodiments, the one or more reference markers disposed at the end of the mechanical arm 152 and the one or more reference markers on the target object need to remain within a target field of view of the navigation device 151, and the target field of view may be an entire field of view of the navigation device 151 or a region within the field of view of the navigation device 151. By determining the target field of view, the navigation device 151 may obtain a pose of the end effector of the mechanical arm 152 at all times, thereby avoiding the end effector of the mechanical arm 152 exceeding the receptive field of the navigation device 151 and resulting a registration failure. The processes disclosed in the present disclosure solve the problem that the space registration of the surgical robot 150 before surgery may easily cause the end effector of the mechanical arm 152 (e.g., the one or more reference markers) to exceed the receptive field of the navigation device 151 during the surgery, and the end effector of a mechanical arm 152 of the surgical robot 150 remains within the field of view of the navigation device 151, thereby improving a success rate of registration.

As shown in FIG. 1, in some embodiments, before performing the surgery on a target object on the support device 153 of the target object (e.g., the scanned bed, the surgical bed, etc.), a space registration of the mechanical arm 152 needs to be performed under the receptive field of the navigation device 151. The target object on the support device 153 of the target object includes but is not limited to, the head, the limb, the abdomen, or the like, and a sensor that is capable of capturing by the navigation device is fixed on a surface of the target object. The navigation device y includes a fixing device, and a dashed region 1511 represents a receptive field (which also be referred to as an initial receptive field) of the navigation device 151. The mechanical arm 152 includes an end effector, and the end effector may be a sensor that is capable of capturing a three-dimensional position by the navigation device, a coordinate system of the end effector of the mechanical arm may be established based on the three-dimensional position.

In some embodiments, the pose of the navigation device 151 may be adjusted based on the target field of view. The pose of the navigation device 151 may be automatically adjusted without relying on manual experience to adjust, improving the accuracy and efficiency of adjusting the pose of the navigation device 151.

In some embodiments, the space registration pose of the surgical robot 150 may be determined based on the target field of view and the pose of the end effector of the mechanical arm of the surgical robot. The space registration pose may be determined based on the target field of view and the pose of the end effector of the mechanical arm of the surgical robot, which can avoid the end effector of the mechanical arm 152 exceeding the receptive field of the navigation device 151 and resulting in a registration failure, and improve the success rate of the registration.

It should be noted that the above descriptions are merely provided for the purpose of illustration and are not intended to limit the scope of the present disclosure. For those of ordinary skill in the art, a variety of amendments and variations may be made under the teaching of the present disclosure. The features, structures, methods, and other characteristics of the exemplary embodiments described in the present disclosure may be combined in various manners to obtain additional and/or alternative exemplary embodiments.

In some embodiments, it is usually necessary for an operator to adjust the pose of the navigation device to a desired state based on experience, and when the positioning of the navigation device is inappropriate, the site to be operated of the end effector of the mechanical arm and/or the site to be operated of the target object are often located outside the field of view of the navigation device, which is not possible to realize a real-time detection and tracking for the site to be operated of the end effector of the mechanical arm and/or the site to be operated of the target object. Therefore, it is desirable to provide a method for determining the space registration pose of the surgical robot to determine a relatively great pose of the navigation device, thereby improving the accuracy and efficiency of the space registration of the surgical robot.

FIG. 2 is a flowchart illustrating an exemplary method for determining a space registration pose of a surgical robot 200 according to some embodiments of the disclosure. As shown in FIG. 2, the process 200 includes the following operations. In some embodiments, the process 200 may be performed by the processing device 110.

In 210, a target field of view of a navigation device is determined. In some embodiments, operation 210 may be performed by the range determination module.

The target field of view refers to a space where the end effector of the mechanical arm may move during the pose registration. To obtain the poses of the site to be operated of the end effector of the mechanical arm and/or the site to be operated of the target object, the movement of the one or more reference markers disposed at the end of the mechanical arm and on the site to be operated of the target object on the support device of the target object needs to be constrained within the target field of view, such that the poses of the site to be operated of the end effector of the mechanical arm and/or the site to be operated of the target object may be obtained by the navigation device at any one of movement moments.

In some embodiments, the target field of view may also be referred to as a second receptive field.

In some embodiments, the range determination module may determine the target field of view of the navigation device in any manner. For example, the target field of view of the navigation device may be determined manually (e.g., by the doctor, etc.).

In some embodiments, the range determination module may obtain an initial receptive field of the navigation device, obtain a space range constraint, and obtain a target field of view of the navigation device by constraining the initial receptive field according to the space range constraint. More descriptions of obtaining the target field of view of the navigation device by constraining the initial receptive field according to the space range constraint may be found in FIG. 3 and the related descriptions thereof, which may not be repeated herein.

In 220, the pose of the navigation device is determined based on the target field of view. In some embodiments, 220 may be performed by the pose determination module.

In some embodiments, the pose determination module may adjust the pose of the navigation device based on the target field of view to make the one or more reference markers of the surgical robot be located within the target field of view.

In some embodiments, the pose determination module may adjust the pose of the navigation device based on the target field of view in any manner. For example, the pose of the navigation device may be adjusted manually based on the target field of view.

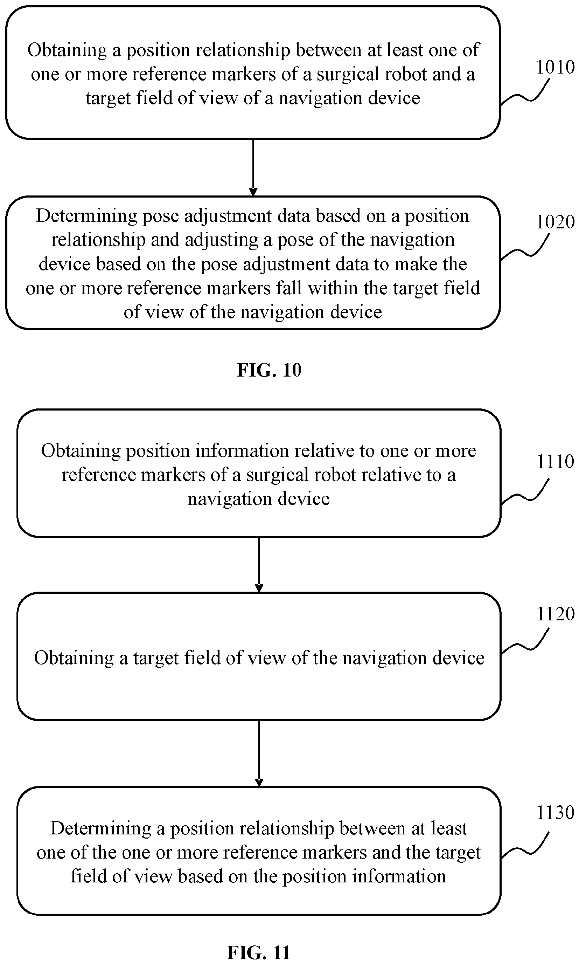

In some embodiments, the pose determination module may obtain a position relationship between the one or more reference markers of the surgical robot and the target field of view of the navigation device, generate pose adjustment data based on the position relationship, and adjust the pose of the navigation device based on the pose adjustment data to make the at least one of one or more reference markers fall within the target field of view of the navigation device. More descriptions of adjusting the pose of the navigation device based on the position relationship between the at least one of the one or more reference markers of the surgical robot and the target field of view of the navigation device may be found in FIG. 10 and the related descriptions thereof, which may not be repeated herein.

In 230, a space registration pose of the surgical robot is determined based on the target field of view and the pose of the end effector of the mechanical arm of the surgical robot. In some embodiments, operation 230 may be performed by the pose registration module.

The space registration pose of the surgical robot is a pose of the end effector of the mechanical arm of the surgical robot for performing the space registration. The space registration pose of the surgical robot may include a position coordinate and a rotation posture of the end effector of the mechanical arm of the surgical robot. The position coordinate may be a coordinate under a certain coordinate system of the surgical robot. For example, the position coordinate may be a coordinate under a navigation device coordinate system, which may be denoted by (x, y, z) . As another example, the position coordinate may also be a coordinate under another coordinate system of the surgical robot (e.g., a mechanical arm coordinate system, a coordinate system of the end of the mechanical arm, and a coordinate system of the end sensor of the mechanical arm, etc.). The rotation posture is an angle or a direction of the end effector of the mechanical arm relative to a coordinate axis at the position coordinate, which may be expressed by an equivalent rotation vector, Euler angle, rotation matrix, posture quaternion, or the like.

The end effector of the mechanical arm of the surgical robot is always located within the target field of view during the space registration of the surgical robot according to the space registration pose.

In some embodiments, the processor of the surgical robot may determine at least one registration path within the target field of view according to the target field of view and the pose of the end effector of the mechanical arm based on a path planning algorithm. The path planning algorithm may be a Lifelong Planning A (*LPA*) algorithm, a D* Lite algorithm, or the like, and the registered path may include a plurality of position points, each position point corresponds to a space registration pose. The pose registration module may control the end effector of the mechanical arm to move along the registration path, determine a space coordinate transformation relationship of at least one component of the surgical robot (e.g., the end effector of the mechanical arm, the target object, the navigation device, etc.), and complete the space registration of the surgical robot.

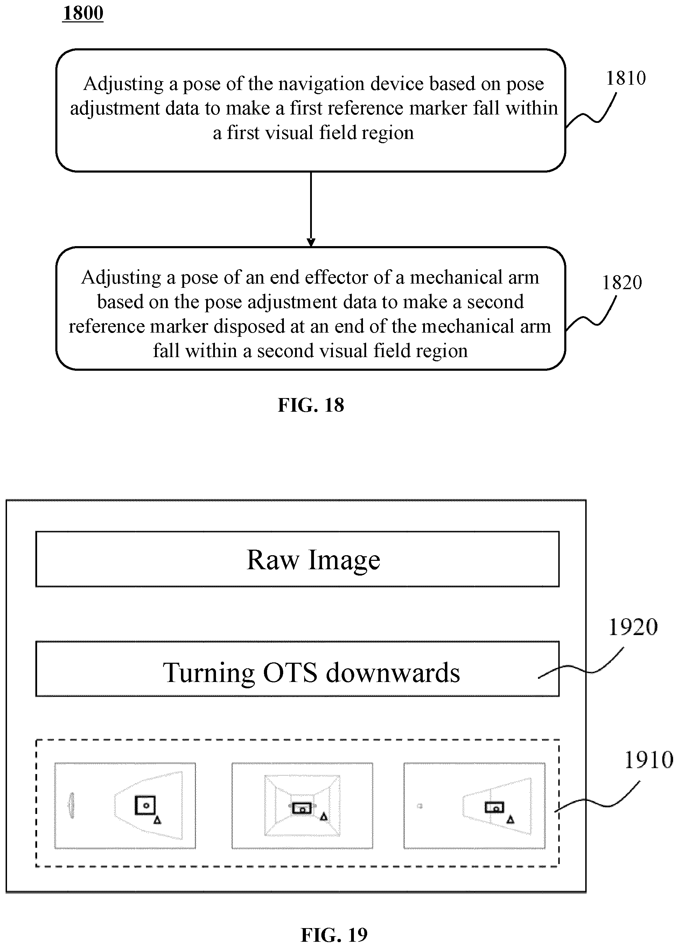

In some embodiments, the pose of the end effector of the mechanical arm of the surgical robot for determining the space registration pose of the surgical robot may be a pre-set initial pose of the end effector of the mechanical arm of the surgical robot; and may also be a pose of the mechanical arm of the surgical robot after adjusting the pose based on the target field of view. More descriptions of the adjusting the pose of the end effector of the mechanical arm based on the target field of view may be found in FIG. 18 and the related descriptions thereof, which may not be repeated herein.

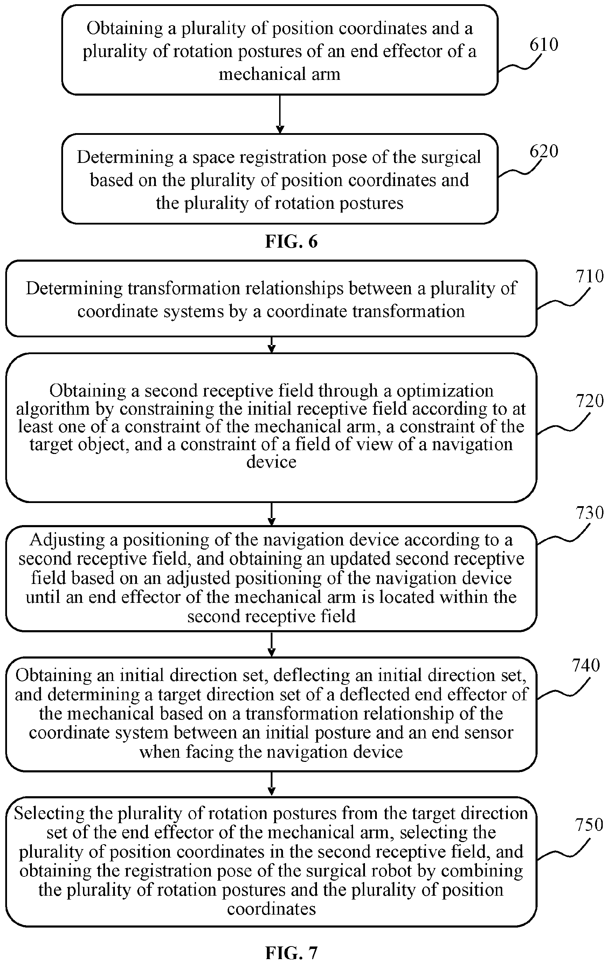

In some embodiments, the pose registration module may obtain a plurality of position coordinates and a plurality of rotation postures of the end effector of the mechanical arm of the surgical robot, and determine the space registration pose of the surgical robot based on the plurality of position coordinates and the plurality of rotation postures. More descriptions of determining the space registration pose of the surgical robot based on the plurality of position coordinates and the plurality of rotation postures may be found in FIG. 6 and its related descriptions, which may not be repeated herein.

In some embodiments, by determining the target field of view of the navigation device, adjusting the pose of the navigation device based on the target field of view, and determining the space registration pose of the surgical robot according to the target field of view and the pose of the end effector of the mechanical arm of the surgical robot, the end effector of the mechanical arm of the surgical robot is avoiding from being located outside the target field of view of the navigation device and resulting in the registration failure, at the same time, making the end effector of the mechanical arm of the surgical robot be located within a relatively great field of view (i.e., a target field of view) of the navigation device to improve registration accuracy.

It should be noted that the foregoing description of process 200 is merely provided for the purpose of example and illustration and is intended to limit the scope of disclosure of the present disclosure. For those skilled in the art, various amendments and variations may be made to the process 200 under the teaching of the present disclosure. However, these amendments and variations remain within the scope of the present disclosure.

During the surgery process, the surgical robots are usually used to assist in completing the surgery, therefore the space registration needs to be performed on the surgical robot before operating the surgery. The purpose of performing the space registration on the surgical robot is to obtain a multi-space coordinate conversion relationship of the end effector of the mechanical arm, the target object, the navigation device, etc., and the coordinate conversion relationship is a prerequisite for normal operation of the surgical robot.

In some embodiments, the process for the space registration of the surgical robot is controlling the mechanical arm of the surgical robot to move according to a designated registration track within the receptive field of the navigation device before the formal surgery to realize the space registration. However, during the surgical process, the receptive field of the navigation device needs to be adjusted accordingly, and a relative position relationship between the navigation device and the base of the mechanical arm also changes. In response to that the relative position relationship between the navigation device and the base of the mechanical arm changes, if the registration track of the mechanical arm is not adjusted accordingly, the sensor of the end effector of the mechanical arm may exceed the receptive field of the navigation device, resulting in a failure of the space registration of the surgical robot and affecting the surgical process.

In some embodiments, the initial receptive field may be adjusted based on the initial receptive field through a space range constraint related to a range of movement of the mechanical arm to obtain a second receptive field that makes the navigation device obtain the position of the mechanical arm at all times, thereby avoiding the end effector of the mechanical arm exceeding the receptive field navigation device and resulting in the registration failure. The present disclosure solves the problem in the related art that the space registration is performed on the surgical robot before the surgery, which easily causes the sensor of the end effector of the mechanical arm to exceed the receptive field of the navigation device during the surgery, and realizes that the end effector of the mechanical arm of the surgical robot remains within the field of view of the navigation device, thereby improving the success rate of the registration.

FIG. 3 is a flowchart illustrating an exemplary process for determining an exemplary target field of view of a navigation device according to some embodiments of the disclosure. As shown in FIG. 3, the process 300 includes the following operations. In some embodiments, the process 300 may be performed by the processing device 110.

In the process of surgery, the surgical robots are usually used to assist in completing the surgery, before the surgery, the space registration needs to be performed on the surgical robot. The purpose of performing the space registration on the surgical robot is to obtain the multi-space coordinate conversion relationship of the end effector of the mechanical arm, the target object, the navigation device, or the like, and the coordinate conversion relationship is a prerequisite for the normal operation of the surgical robot.

In some embodiments, the mechanical arm of the surgical robot is controlled to move within a receptive field of the navigation device according to a designated registration track to realize the space registration, which easily causes the problem that the end effector of the mechanical arm (e.g., the sensor) exceeds the receptive field of the navigation device during the surgery.

In some embodiments, the process 300 adjusts the initial receptive field based on the initial receptive field through the space range constraint related to the range of movement of the mechanical arm to obtain a target field of view that makes the navigation device obtain the position of the mechanical arm at all times, thereby avoiding the end effector of the mechanical arm exceeding the receptive field of the navigation device and resulting in the registration failure. The foregoing processes make the end effector of the mechanical arm of the surgical robot remain within the field of view of the navigation device and improve the success rate of the registration.

In 310, the initial receptive field of the navigation device is obtained. In some embodiments, operation 310 may be performed by the range determination module.

The initial receptive field is a field of view that is obtained by the navigation device. In some embodiments, the initial receptive field may further be referred to as a first receptive field. It should be understood that the initial receptive field may be determined based on the hardware characteristics of the navigation device. For example, the initial receptive field of the navigation device may be an initial receptive field determined by factory settings in the navigation device. In some embodiments, the surgical robot is configured to perform the surgery on the target object, and the navigation device is configured to obtain a position of the end effector of the mechanical arm of the surgical robot and a position of the target object to match the position of the end effector of the mechanical arm with the position of the target object to improve the accuracy of the anatomical position recognition. A common navigation device includes an optical navigation device, an electromagnetic navigation device, or the like. Specifically, the optical navigation device includes a binocular optical navigation device, a trinocular optical navigation device, a structural optical navigation device, or the like.

In some embodiments, the range determination module may obtain the initial receptive field of the navigation device from the processing device 110, the user terminal 130, the storage device 140, the surgical robot 150, and/or the external data source.

In 320, the space range constraint is obtained, and the target field of view of the navigation device is obtained by constraining the initial receptive field according to the space range constraint. In some embodiments, operation 320 may be performed by the range determination module.

Since the mechanical arm needs to move during the registration, the position of the end effector of the mechanical arm is located outside the initial receptive field. A second receptive field (i.e., the target field of view) with an optimal space position is obtained by adjusting the initial receptive field based on the initial receptive field in this embodiment. Specifically, the initial receptive field is constrained based on the space range constraint.

The space range constraint may be a condition for limiting the movable space of the end effector of the mechanical arm, which may be pre-stored in the processor of the navigation device. The detailed content of the space range constraint may be set by a staff member based on experience or may be obtained by training with a model such as a neural network to obtain the optimal space range constraint. The space range constraint of the mechanical arm may be related to a relative position relationship between the end effector of the mechanical arm and the site to be operated of the target object, such as a direction of the end effector of the mechanical arm relative to the site to be operated of the target object, a shortest distance between the end effector of the mechanical arm and the site to be operated of the target object, a range of a tilt angle of the end effector of the mechanical arm relative to the site to be operated of the target object, or the like. The constraint may be related to a relative position relationship between the end effector of the mechanical arm and the navigation device, such as the direction of the end effector of the mechanical arm relative to the navigation device, the shortest distance between the end effector of the mechanical arm and the navigation device, or the like. The constraint may further include other constraints of the movement of the end effector of the mechanical arm, such as the shortest distance between the end effector of the mechanical arm and other devices (e.g., the support device 153 of the target object, etc.), or the like.

In some embodiments, the range determination module may obtain the space range constraint from the processing device 110, the user terminal 130, the storage device 140, the surgical robot 150, and/or the external data source.

In some embodiments, the space range constraint may be set by a staff member based on experience or may be obtained by training through a model (e.g., a neural network model) to obtain an optimal space range constraint.

In some embodiments, the space range constraint may include at least one of a constraint of the mechanical arm, a constraint of the target object, and a constraint of the field of view of the navigation device.

The constraint of the mechanical arm may characterize a reachable range of the mechanical arm, and the constraint of the mechanical arm may include the length of each joint of the mechanical arm, a rotatable angle of each joint of the mechanical arm, or the like. In some embodiments, the range determination module may obtain a reachable range of the mechanical arm through a variety of sensors, for example, the range determination module may obtain the length of each joint of the mechanical arm based on a range sensor and obtain the rotatable angle of each joint of the mechanical arm based on a corner sensor. In some embodiments, the reachable range of the mechanical arm may be determined by various types of sensors of the optical navigation device. For example, the various types of sensors may include at least an end sensor of the mechanical arm, an end sensor of the target object, the various types of sensors may further include a sensor of the reference position, or the like.

In some embodiments, the reachable range of the mechanical arm may further be determined based on conditions such as a limiting constraint of the mechanical arm, a singular configuration constraint, and a self-occlusion of the mechanical arm. The limiting constraint of the mechanical arm may characterize a range of an angle at which each joint of the mechanical arm may be allowed to rotate, and the limiting constraint of the mechanical arm may include a range of safe rotation angles for each joint of the mechanical arm. The singular configuration constraint may characterize a pose that avoids not allowing the mechanical arm to be placed, and the singular configuration constraint may include at least one pose of a mechanical arm corresponding to a singular configuration. The self-occlusion of the mechanical arm may characterize a disallowed pose that the mechanical arm occludes the end effector of the mechanical arm, and the self-occlusion of the mechanical arm may include a pose where the mechanical arm occludes the end effector of the mechanical arm, a pose where the mechanical arm occludes the site to be operated of the target, or the like.

The constraint of the target object may characterize a safety distance between the end effector of the mechanical arm and the target object, and the constraint of the target object may include the shortest distance between the end effector of the mechanical arm and the site to be operated of the target object, the end effector of the mechanical arm being located in a sterilized region or the like. The constraint of the target object may be determined by the safe distance of the end effector of the mechanical arm from the target object.

The constraint of the field of view of the navigation device may be a high precision localization range of the navigation device. It should be understood that the high-precision localization range of the navigation device may be a portion of the initial receptive field. For example, the high-precision localization range of the navigation device may be an intermediate region of the initial receptive field. The constraint of the field of view of the navigation device may construct an effective range of the receptive field of the optical navigation device through a mechanical arm configuration, or the like.

In some embodiments, electromagnetic interference further needs to be considered if the initial receptive field of the electromagnetic navigation device needs to be constrained.

The initial receptive field is constrained based on the space range constraint to obtain the target field of view of the navigation device, such that various positions of the second receptive field (i.e., the target field of view of the navigation device) satisfy all the constraints. In some embodiments, the second receptive field may be determined based on at least one of the constraint of the mechanical arm, the constraint of the target object, and the field of view of the navigation device constraint, which can improve the safety of the surgical robot during registration.

In some embodiments, the range determination module may obtain the target field of view of the navigation device in any feasible manner that constrains the initial receptive field of view according to the space range constraint.

Merely by way of example, the range determination module may obtain the target field of view of the navigation device through a range determination model that constrains the initial receptive field based on the space range constraint. The range determination model is a machine learning model for determining the target field of view. An input of the range determination model may include the space range constraint (e.g., at least one of the constraint of the mechanical arm, the constraint of the target object, and the constraint of the field of view of the navigation device, etc.). An output of the range determination model may include the target field of view of the navigation device. The range determination model may include a convolutional neural network (CNN), a recurrent neural network (RNN), a multilayer neural network (MLP), an adversarial neural network (GAN), or any combination thereof. In some embodiments, the range determination module may update the parameters of the range determination module by a plurality of sets of training samples to obtain a trained range determination model. The training samples may include a space range constraint of a sample surgical robot, and a label of the training samples refers to a target field of view corresponding to the sample surgical robot.

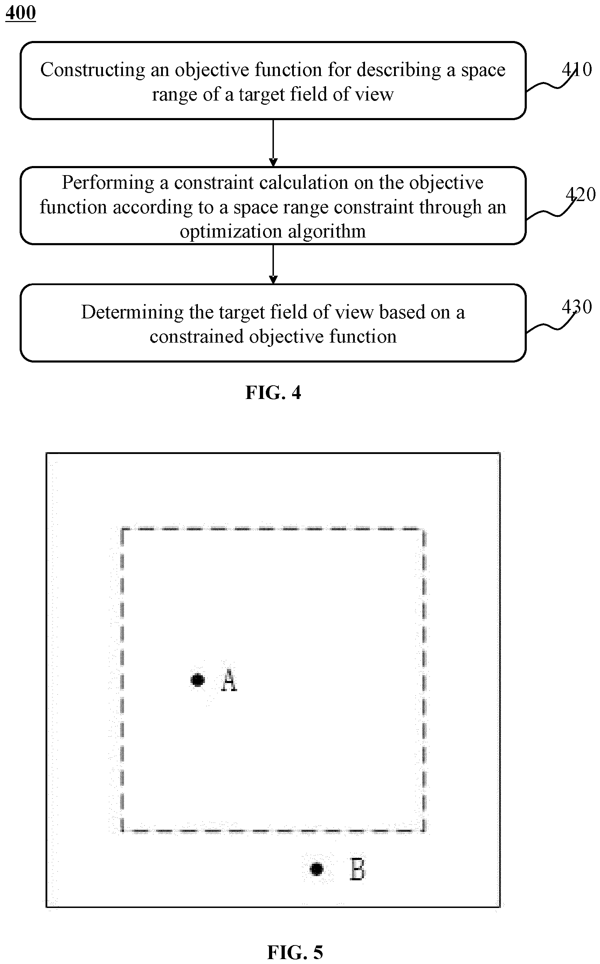

In some embodiments, the space range constraint may construct an objective function for describing a space range of the target field of view, perform a constraint calculation on the objective function based on the space range constraint through an optimization algorithm, and determine the target field of view based on a constraint calculation on the objective function. More descriptions of determining the target field of view based on the objective function for describing the space range of the target field of view may be found in FIG. 4 and the related description, which may not be repeated herein.

In some embodiments, the space range constraint is obtained, and the target field of view of the navigation device is obtained by constraining the initial receptive field according to the space range constraint, which may make the end effector of the mechanical arm and the site to be operated of the target object on the support device of the target object always be located within the target field of view when the surgical robot performs the space registration subsequently, so that the navigation device may obtain the pose of the site to be operated of the target object, thereby avoiding a failure of the space registration of the surgical robot.

In some embodiments, process 300 may further include operation 330. In 330, after the target field of view of the navigation device is determined, the positioning of the navigation device is adjusted according to the target field of view to make the end effector of the mechanical arm of the surgical robot located within the target field of view. In some embodiments, operation 330 may be performed by the range determination module.

In some embodiments, after obtaining the second receptive field of the navigation device, the positioning of the navigation device may further be adjusted according to the second receptive field to make the end effector of the mechanical arm of the surgical robot be located within the second receptive field. In some embodiments, the positioning may be automatically realized by a motor, or realized manually by the staff member. FIG. 5 is a schematic diagram illustrating a receptive field according to some embodiments of the present disclosure. As shown in FIG. 5, a solid line box is the initial receptive field of the navigation device, a dashed line box is a second receptive field after being constrained by the space range constraint, a sensor A is an end effector of a mechanical arm, and a sensor B obtains a target object sensor of a position of the target object. Typically, sensor A is located within the second receptive field indicated by the dashed line box to avoid sensor A exceeding the actual receptive field of the navigation device when the space registration is performed on the surgical robot. If sensor A is not located within the second receptive field, the positioning of the navigation device (e.g., an angle and the pose of the navigation device) may be adjusted to adjust the position of the second receptive field to make sensor A be located within the second receptive field, thereby ensuring that the end effector of the mechanical arm is located within the second receptive field, and improving the success rate of the registration of the surgical robot.

Further, if the end effector of the mechanical arm is not within the second receptive field, the positioning of the navigation device may be adjusted and the second receptive field is obtained again according to the space range constraint until the end effector of the mechanical arm is located in the second receptive field.

In some embodiments, after determining the target field of view of the navigation device, the positioning of the navigation device is adjusted according to the target field of view to make the end effector of the mechanical arm of the surgical robot located within the target field of view, which may cause the end effector of the mechanical arm of the surgical robot always be located within the target field of view when the space registration is performed on the surgical robot subsequently, and avoiding the registration failure.

In some embodiments, a target field of view 300 of the navigation device is determined, and by obtaining the initial receptive field of the navigation device and the space range constraint of the navigation device, the initial receptive field is constrained based on the space range constraint, the target field of view of the navigation device may be determined quickly and accurately.

It should be noted that the foregoing description of process 300 is merely provided for the purpose of example and illustration and is not intended to limit the scope of disclosure of the present disclosure. For those skilled in the art, various amendments and variations may be made to the process 800 under the teaching of the present disclosure. However, these amendments and variations remain within the scope of the present disclosure.

FIG. 4 is a flowchart illustrating an exemplary process for obtaining a target field of view of a navigation device by constraining an initial receptive field according to a space range constraint according to some embodiments of the disclosure. As shown in FIG. 4, the process 400 includes the following operations. In some embodiments, the process 400 may be performed by the processing device 110.

In 410, an objective function for describing a space range of the target field of view is constructed, operation 410 may be performed by the range determination module.

The objective function is a function for describing a space range corresponding to the target field of view. In some embodiments, an objective function may be expressed in any form. For example, the objective function may be expressed as max. H(f(x, y, z)), wherein x, y, z ∈ R, and R is a set of real numbers. f(x, y, z) denotes a space range corresponding to the target field of view, and max.H( ) denotes a maximum effective solution for the space range corresponding to the target field of view.

In 420, a constraint calculation is performed on the objective function according to the space range constraint through an optimization algorithm. In some embodiments, operation 420 may be performed by the range determination module.

The optimization algorithm is a process of describing a qualitative or quantitative mathematical problem by constructing an appropriate mathematical model and designing an appropriate computational approach to find an optimal solution for the mathematical model. The optimization algorithm may include linear programming, semidefinite programming, least squares problems, composite optimization, matrix optimization, stochastic optimization, gradient descent, Newton's method and Quasi-Newton's method, conjugate gradient method, heuristic optimization methods, or the like.

In some embodiments, the range determination module may solve the objective function by performing a constraint calculation on the objective function according to the space range constraint through a typical optimization software program for a language (e.g., MATLAB or Python).

In 430, the target field of view is determined based on the constrained objective function. In some embodiments, operation 430 may be performed by the range determination module.

In some embodiments, the range determination module may designate a result of the constrained objective function as the target field of view.

In some embodiments, by constructing the objective function for describing the space range of the target field of view and performing a constraint calculation on the objective function based on the space range constraint through the optimization algorithm, a relatively more accurate target field of view may be quickly determined based on the constrained objective function.

FIG. 6 is a flowchart illustrating an exemplary process for determining a space registration pose of a surgical robot according to some embodiments of the disclosure. As shown in FIG. 6, the process 600 includes the following operations. In some embodiments, the process 600 may be performed by the processing device 110.

In 610, a plurality of position coordinates and a plurality of rotation postures of the end effector of the mechanical arm are obtained. In some embodiments, operation 610 may be performed by the pose registration module.

A position coordinate may include at least one corresponding rotation posture. In some embodiments, the count of rotation postures corresponding to different position coordinates may be different. For example, a count of rotation postures corresponding to the position coordinates that are closer to the site to be operated of the target object may be greater than a count of rotation postures corresponding to the position coordinates that are further away from the site to be operated of the target object.

The position coordinate refers to a space position coordinate under a navigation device coordinate system, expressed as (x, y, z). When a transformation relationship between the navigation device coordinate system and other coordinate systems may be obtained, the position coordinate may further be expressed as another position coordinate under another coordinate system, such as a mechanical arm coordinate system, a coordinate system of the end of the mechanical arm, and a coordinate system of the end sensor of the mechanical arm. The rotation posture represents an angle or a direction of the end effector of the mechanical arm relative to a coordinate axis under the space position, which may be expressed by an equivalent rotation vector, a Euler angle, a rotation matrix, a posture quaternion, or the like. The plurality of rotation postures form a target direction set for the end effector of the mechanical arm.

In some embodiments, the pose registration module may obtain the plurality of position coordinates and the plurality of rotation postures from the processing device 110, the user terminal 130, the storage device 140, the surgical robot 150, or the external data source.

In some embodiments, the pose registration module may obtain the plurality of initial rotation postures of the end effector of the mechanical arm, and obtain the plurality of rotation postures by deflecting the plurality of initial rotation postures in the same direction.

The plurality of initial rotation postures refer to be preset rotation postures, and the plurality of initial rotation postures are capable of forming an initial direction set for the end effector of the mechanical arm.

In some embodiments, the pose registration module may obtain a plurality of initial rotation postures from the processing device 110, the user terminal 130, the storage device 140, the surgical robot 150, or the external data source.

In some embodiments, the pose registration module may adjust an initial rotation posture of the end effector of the mechanical arm to generate a plurality of initial rotation postures.

In some embodiments, the pose registration module may obtain a maximum posture range of the end effector of the mechanical arm, and determine the plurality of initial rotation postures of the end effector of the mechanical arm in the maximum posture range according to a count of postures and/or a posture dispersion of the end effector of the mechanical arm.

When obtaining the initial direction set, the maximum posture range of the end effector of the mechanical arm identified by the navigation device needs to be obtained first. In some embodiments, the maximum posture range may be obtained by the staff member detecting in conjunction with characteristics of the navigation device. In some embodiments, the pose registration module may obtain the maximum posture range from the processing device 110, the user terminal 130, the storage device 140, the surgical robot 150, or the external data source. To make the rotation posture of the surgical robot maximally cover all rotation postures of the mechanical arm when performing the space registration, the posture dispersion is set herein. The posture dispersion represents a space-relative posture of two neighboring rotation postures. For example, if an angel of a rotation posture relative to the coordinate axis is 10° and an angel of another rotation posture relative to the coordinate axis is 30°, and the posture dispersion is 20°. An initial direction set of the end effector of the mechanical arm is determined based on the count of postures and/or the posture dispersion of the end effector of the mechanical arm under the maximum posture range. The count of postures may be a total count of different postures of the end effector of the mechanical arm under the second receptive field.

For example, the pose registration module may determine a plurality of initial rotation postures of the end effector of the mechanical arm based on the count of postures of the end effector. Merely by way of example, when the count of postures is 7, 5 initial rotation postures close to the site to be operated of the target object and 2 initial rotation postures of the site to be operated of the target object away from the target object may be determined within the maximum posture range of the end effector of the mechanical arm.

As another example, when the maximum posture range is obtained, the pose registration module may first obtain the count of postures and distribute the count of rotation postures to the target field of view. Merely by way of example, the maximum posture range is set to [-45°, 45°], when the count of postures is five, one of the plurality of rotation postures may be set every 22.5°, and 22.5° is be a corresponding posture dispersion, and the plurality of rotation postures may further be set densely in a space range close to the target object and set sparsely in a space range away from the target object, such as -45°, -22.5°, 0°, 22.5°, 45°.

As another example, the pose registration module may first determine the posture dispersion and determine specific rotation postures within the target field of view based on the maximum posture range. Merely by way of example, a posture dispersion is set to 15° in a maximum posture range of [-45°, 45°], and there are a total of seven rotation postures.

Further, when the posture range is not informed, the pose registration module may jointly determine the initial direction set according to the count of postures and the rotation postures. Taking a binocular optical navigation device as an example, the sensor disposed on the end effector of the mechanical arm may be identified by the binocular optical navigation device when the mechanical arm is in a discrete posture.

In some embodiments, the pose registration module may obtain a plurality of rotation postures by deflecting the plurality of initial rotation postures in the same direction. The deflection includes a deflection along a direction close to the navigation device, if there is a deformity of the mechanical arm or the mechanical arm is not reachable, the plurality of initial rotation postures are deflected close to a direction toward the navigation device based on the initial direction.

In some embodiments, the pose registration module needs to obtain transformation relationships between various coordinate systems in the surgical robot to realize transformations of the position coordinates and the rotation postures in different coordinate systems. The coordinate systems of the surgical robot include at least a portion of the following coordinate systems: the mechanical arm coordinate system, the navigation device coordinate system, the coordinate system of the end of the mechanical arm, and the coordinate system of the end sensor of the mechanical arm. More descriptions of determining the registration pose of the surgical robot based on the transformation relationships between the coordinate systems in the surgical robot may be found in FIG. 7 and the related descriptions, which may not be repeated herein.

In some embodiments, by obtaining the plurality of initial rotation postures of the end effector of the mechanical arm and deflecting the plurality of initial rotation postures in the same direction, the plurality of rotation postures may be quickly obtained. Moreover, the rotation postures within the second receptive field may be ensured to be more evenly distributed, providing more options for the surgical robot to be registered.

In 620, the space registration pose of the surgical robot is determined based on the plurality of position coordinates and the plurality of rotation postures. In some embodiments, operation 620 may be performed by the pose registration module.

In some embodiments, the position coordinates and the rotation postures are non-strongly correlated, and the position coordinates may be selected in the second receptive field. The options of the rotation postures are not limited by the second receptive field and may be selected either within the second receptive field or outside the second receptive field. Finally, the space registration pose may be obtained by combining the selected position coordinates and the selected rotation postures.

In some embodiments, the pose registration module may set a plurality of corresponding rotation postures for each position coordinate, and the correspondences may be set in advance. For example, when there are the plurality of position coordinates, for each position coordinate close to the target object or the scanning bed, the plurality of rotation postures may be set to obtain a more accurate registration track, and for the position coordinates far away from the target object or the scanning bed, the count of rotation postures corresponding to the position coordinates may be less. Moreover, for a space range close to the target object or the scanning bed, denser position coordinates may be set for the space registration of the surgical robot.

It should be noted that the foregoing description of process 600 is merely provided for the purpose of example and illustration and is not intended to limit the scope of disclosure of the present disclosure. For those skilled in the art, various amendments and variations may be made to the process 600 under the teaching of the present disclosure. However, these amendments and variations remain within the scope of the present disclosure.

FIG. 7 is a flowchart illustrating an exemplary process for determining a registration pose of a surgical robot based on a transformation relationship between various coordinate systems in a surgical robot according to some embodiments of the disclosure. As shown in FIG. 7, the process 700 includes the following operations. In some embodiments, the process 700 may be performed by the processing device 110.

In 710, transformation relationships between the plurality of coordinate systems are determined by a coordinate transformation. In some embodiments, operation 710 may be performed by the pose registration module.

The plurality of coordinate systems may include a coordinate system of an end joint of the mechanical arm, a coordinate system of the end effector of the mechanical arm, a coordinate system of a mechanical arm base of the navigation device coordinate system, or the like. In some embodiments, the pose registration module may solve the transformation relationships between the coordinate systems based on coordinates obtained by the end sensor of the mechanical arm, coordinates of the initialized poses of the mechanical arm, and coordinates of the end joint of the mechanical arm, the end sensor is a sensor disposed on the end effector of the mechanical arm.

In 720, the second receptive field is obtained through the optimization algorithm by constraining the initial receptive field according to at least one of the constraint of the mechanical arm, the constraint of the target object, and the constraint of the field of view of the navigation device. In some embodiments, the operation 720 may be performed by the pose registration module.

More descriptions of obtaining the second receptive field through the optimization algorithm by constraining the initial receptive field according to at least one of the constraint of the mechanical arm, the constraint of the target object, and the constraint of the field of view of the navigation device may be found in FIG. 3 and FIG. 4, and the related descriptions, which are not be repeated herein.

In some embodiments, the constraint of the target object is determined based on the coordinates obtained by the sensor of the target object.

In 730, the positioning of the navigation device is adjusted according to the second receptive field, and an updated second receptive field is obtained based on the adjusted positioning of the navigation device until the end effector of the mechanical arm is located within that second receptive field. In some embodiments, the operation 730 may be performed by the pose registration module.

More descriptions of adjusting the positioning of the navigation device according to the second receptive field may be found in FIG. 3 and the related description, which may not be repeated herein.

In 740, the initial direction set is obtained, the initial direction set may be deflected, and a target direction set of the deflected end effector of the mechanical arm may be determined based on a transformation relationship of the coordinate system between the initial posture and the end sensor when facing the navigation device. In some embodiments, operation 740 may be performed by the pose registration module.

In some embodiments, the coordinate of the end effector of the mechanical arm tracked by the navigation device is designated as the initial position coordinate, and the initial coordinate system transformation relationship between the end effector of the mechanical arm and the navigation device is determined based on the initial position coordinate in conjunction with a theoretical coordinate relationship between the end effector of the mechanical arm and the end joint of the mechanical arm, and at this time, the pose of the mechanical arm may be used as an initial posture of the mechanical arm Porigin. In addition, a coordinate system relationship between the mechanical arm and the navigation device needs to be determined when the end sensor faces the navigation device.

In 750, the plurality of rotation postures are selected from the target direction set of the end effector of the mechanical arm, the plurality of position coordinates are selected in the second receptive field, and the registration pose of the surgical robot is obtained by combining the plurality of rotation postures and the plurality of position coordinates. In some embodiments, operation 750 may be performed by the pose registration module.

The pose is represented by (x, y, z, rx, ry, rz), wherein x, y, z denotes a position coordinate, and rx, ry, rz denotes a rotation posture. After the pose is obtained, the pose registration module may determine an angle of each joint in the mechanical arm by kinematic inverse solution.

In some embodiments, the target direction set is primarily configured to determine a rotation posture (rx, ry, rz) of the end effector of the mechanical arm corresponding to each registration pose in pose determination, and the determination of the space position is configured to determine a space position (x, y, z) of each registration pose.

In some embodiments, the process 700 adjusts the initial receptive field based on the initial receptive field through a space range constraint related to a movement range of the mechanical arm to obtain the second receptive field that makes the navigation device able to obtain the position of the mechanical arm at all times, thereby avoiding the end effector of the mechanical arm from exceeding the receptive range the navigation device and resulting in the registration failure. The target object of the target object solves the problem in the related art that the space registration is performed on the surgical robot before the surgery, which easily causes the sensor of the end effector of the mechanical arm to exceed the receptive field of the navigation device during the surgery, and realizes that the end effector of the mechanical arm of the surgical robot remains within the field of view of the navigation device, thereby improving the success rate of the registration.

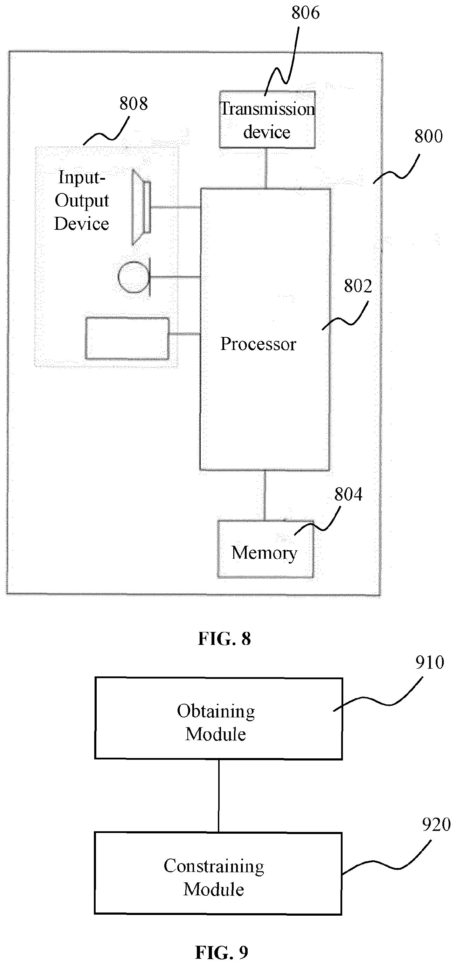

The embodiments provided in the present disclosure may be executed in a terminal, a computer, or a similar computing device. Taking running on the terminal as an example, FIG. 8 is a block diagram illustrating an exemplary hardware structure of a terminal for performing a process for obtaining a visual navigation device receptive field according to some embodiments of the disclosure. As shown in FIG. 8, the terminal 800 may include one or more (only one is shown in FIG. 8) processors 802 (the one or more processors 802 may include but are not limited to one or more processing devices such as a microprocessor MCU or a programmable logic devices FPGA, etc.) and one or more memory 804 for storing data. In some embodiments, the terminal described above may further include a transmission device 806 for a communication function, and an input-output device 808. It should be understood that for those skilled in the art, a structure shown in FIG. 8 is merely schematic, which does not limit the structure of the above terminal. For example, the terminal 800 may further include more or fewer components than the terminal 800 shown in FIG. 8, or has a different configuration from the terminal 800 shown in FIG. 8.

The memory 804 is configured to store control programs, such as software programs and modules of an application software (e.g., a control program corresponding to a process for obtaining a visual navigation device receptive field in the embodiments of the present disclosure). The processor 802 executes various functional disclosures and data processing by running the control programs stored in the memory 804, i.e., realizes the process described above. The memory 804 includes one or more high-speed random memories, and further includes one or more non-volatile memories (e.g., one or more magnetic storage devices, flash memory, or other non-volatile solid-state memory). In some examples, the memory 804 further includes one or more remotely-disposed memories relative to the processor 802, and the remote memories may be connected to the terminal 800 through the network. The above examples of networks include but are not limited to an Internet, an enterprise intranet, a local region network, a mobile communication network, or any combination thereof.

The transmission device 806 is configured to receive or send data through the network. The specific examples of the network described above includes a wireless network provided by a communication provider of the terminal 800. In some embodiments, the transmission device 806 includes a Network Interface Controller (NIC) that is connected to other network devices through a base station, thereby communicating with the Internet. In some embodiments, the transmission device 806 may be a Radio Frequency (RF) module for wirelessly communicating with the Internet.

The embodiments in the present disclosure also provide a surgical robot, including the navigation device, the mechanical arm, and the processor. The processor obtains the initial receptive field of the navigation device. The processor obtains the space range constraint, and obtain the target field of view of the navigation device by constraining the initial receptive field according to the space range constraint. The space range constraint includes at least one of the constraint of the mechanical arm, the constraint of the target object, and the constraint of the field of view of the navigation device.

Further, a specific process for obtaining the second receptive field of the processor may include: constructing an objective function for describing a space range of the second receptive field; performing the constraint calculation on the objective function based on the space range constraint through an optimization algorithm; and determining the second receptive field based on the constrained objective function.

Further, after obtaining the second receptive field, the processor may further adjust the positioning of the navigation device according to the second receptive field to make the end effector of the mechanical arm of the surgical robot be located within the second receptive field.

Further, after obtaining the second receptive field, the processor determines a space registration pose of the surgical robot by combining the second receptive field and the pose of the end effector of the mechanical arm of the surgical robot. Specifically, a process for determining the space registration pose of the surgical robot includes: obtaining the plurality of position coordinates and the plurality of rotation postures of the end effector of the mechanical arm of the surgical robot, wherein the plurality of rotation postures form the target direction set of the mechanical arm of the mechanical arm; and determining the space registration pose of the surgical robot based on the plurality of position coordinates and a plurality of rotation postures of the target direction set, wherein the plurality of position coordinates correspond to the plurality of rotation postures.

Further, a process for obtaining the target direction set of the processor includes: obtaining an initial direction set of the end effector of the mechanical arm, wherein the initial direction set includes the plurality of initial rotation postures; and obtaining the target direction set of the end effector of the mechanical arm by deflecting the plurality of initial rotation postures in the initial direction set in the same direction. The initial direction set is obtained by: obtaining a maximum posture range of the end effector of the mechanical arm identified by the navigation device; and determining the initial direction set of the end effector of the mechanical arm in the maximum posture range based on the count of postures and/or the posture dispersion of the end effector of the mechanical arm.

The surgical robot in some embodiments adjusts the initial receptive field through the space range constraint related to the movement range of the mechanical arm based on the initial receptive field, and obtain the second receptive field that makes the navigation device obtain the position of the mechanical arm at all times, thereby avoiding the end effector of the mechanical arm exceeding the range of the navigation device and resulting in the registration failure. The target object of the target object solves the problem in the related art that the space registration is performed on the surgical robot before the surgery, which easily causes the sensor of the end effector of the mechanical arm to exceed the receptive field of the navigation device during the surgery, and realizes that the end effector of the mechanical arm of the surgical robot remains within the field of view of the navigation device, thereby improving the success rate of the registration.

The present embodiment further provides a device for obtaining a visual navigation device receptive field, and the device is configured to realize the above embodiments and preferred embodiments, which is not repeated as already described. As used hereinafter, the terms "module", "unit", "sub-unit", or the like, may be a combination of software and/or hardware that realizes a preset function. Although the devices described in the following embodiments are preferably implemented in software, the implementation of hardware, or a combination of software and hardware, is also possible and contemplated.

FIG. 9 is a block diagram illustrating an exemplary structure of a device for obtaining a visual navigation device receptive field according to some embodiments of the disclosure. As shown in FIG. 9, the device includes an obtaining module 910 and a constraining module 920. The obtaining module 910 is configured to obtain the initial receptive field of the navigation device in the surgical robot. The constraint module 920 is configured to obtain the space range constraint, and obtain the second receptive field of the navigation device by constraining the initial receptive field according to the space range constraint. The space range constraint includes at least one of the constraint of the mechanical arm, the constraint of the target object, and the constraint of the field of view of the navigation device.

Further, the constraint module 920 is further configured to construct the objective function for describing the space range of the second receptive field, perform the constraint calculation on the objective function based on the space range constraint through the optimization algorithm, and determine the second receptive field based on the constrained objective function.

Further, the device for obtaining the visual navigation device receptive field includes a positioning module, the positioning module is configured to adjust the positioning of the navigation device according to the second receptive field to make the end effector of the mechanical arm of the surgical robot be located within the second receptive field.

Further, the device for obtaining the visual navigation device receptive field includes a registration module, and the registration module is configured to determine the space registration pose of the surgical robot in conjunction with the pose of the end effector of the mechanical arm of the surgical robot in the second receptive field. Specifically, the registration module includes a direction set calculation unit configured to obtain the plurality of position coordinates and the plurality of rotation postures of the end effector of the mechanical arm of the surgical robot, wherein the plurality of rotation postures form the target direction set of the end effector of the mechanical arm, and determine the space registration pose of the surgical robot based on the plurality of position coordinates and the plurality of rotation postures in the target direction set. The plurality of position coordinates correspond to the plurality of the rotation postures, respectively.

Further, the registration module includes a deflection unit, and the deflection unit is configured to obtain an initial direction set of the end effector of the mechanical arm. The initial direction set includes the plurality of initial rotation postures. The target direction set of the end effector of the mechanical arm is obtained by deflecting the plurality of initial rotation postures to the same direction. The initial direction set is obtained by: obtaining the maximum posture range of the end effector of the mechanical arm identified by the navigation device, determining the initial direction set of the end effector of the mechanical arm in the maximum posture range based on the count of postures and/or the posture dispersion of the end effector of the mechanical arm.

For the surgical robot in some embodiments, a constraint module 920 adjusts the initial receptive field through the space range constraint related to the movement range of the mechanical arm based on the initial receptive field obtained by the obtaining module 910, and obtain the second receptive field that makes the navigation device obtain the position of the mechanical arm at all times, thereby avoiding the end effector of the mechanical arm exceeding the receptive field of the navigation device and resulting in the registration failure. The target object of the target object solves the problem in the related art that the space registration is performed on the surgical robot before the surgery, which easily causes the sensor of the end effector of the mechanical arm to exceed the receptive field of the navigation device during the surgery, and realizes that the end effector of the mechanical arm of the surgical robot remains within the field of view of the navigation device, thereby improving the success rate of the registration.

It should be noted that each of the modules described above may be a function module or a program module, and may be implemented either by software or by hardware. For the modules implemented by hardware, each of the modules described above may be located in the same processor; or each of the modules described above may be located in different processors according to the form of any combination.

The embodiment further provides an electronic device including the memory and the processor, the memory stores the computer programs, and the processor is set to operate the computer programs to perform the operations in any of the following method embodiments.

In some embodiments, the above electronic device may further include a transmission device and an input-output device 808, and the transmission device and the input-output device 808 are both connected to the processor.

In some embodiments, the processor may be set to perform the following operations through the computer programs:

In S 1, the initial receptive field of the navigation device in the surgical robot is obtained;

In S2, the space range constraint is obtained, and the second receptive field of the navigation device is obtained by constraining the initial receptive field according to the space range constraint.

In some embodiments, the processor may be set to perform the following operations through the computer programs:

In Y1, the initial receptive field of the navigation device in the surgical robot is obtained;

In Y2, the space range constraint is obtained, and the second receptive field of the navigation device is obtained by constraining the initial receptive field according to the space range constraint;

In Y3, the space registration pose of the surgical robot is determined based on the second receptive field and the pose of the end effector of the mechanical arm of the surgical robot.

It should be noted that specific examples in this embodiment may be referred to the examples described in the above embodiments and optional embodiments, which are not be repeated herein.

FIG. 10 is a flowchart illustrating an exemplary for determining an exemplary target pose of a navigation device based on a target field of view according to some embodiments of the present disclosure. As shown in FIG. 10, the process 1000 includes the following operations. In some embodiments, the process 1000 may be performed by the processing device 110.

Surgical navigation refers to tracking a surgical position in real-time through a three-dimensional digital target object lesion tissue to realize surgical visualization and automation, thereby assisting a doctor in completing a surgical task more quickly, accurately, and safely. Optical navigation is a mainstream process in current surgical navigation, and has the advantages of high precision, flexible and convenient application, no invasive damage, or the like., therefore, the optical navigation device has a wide range of applications, and may detect and track the target object in real-time during surgery by using the optical navigation device, thereby assisting a surgical operation process.

The optical navigation device usually needs to adjust the pose of the optical navigation device before detecting and tracking the target object in real-time. The pose of the optical navigator is generally adjusted to a desired state by the operator based on experience when being adjusted.

However, the above process relying on manual adjustment has low accuracy and efficiency in adjusting the pose of the optical navigation device.

In some embodiments, the process 1000 obtains a position relationship between at least one of one or more reference markers of the surgical robot and a target field of view of the navigation device of the surgical robot; generate pose adjustment data based on the position relationship, and adjusts a pose of the optical navigation device based on the pose adjustment data to make the at least one of the one or more reference markers fall within the target field of view of the navigation device. Process 100 may directly adjust the pose of the navigation device through the pose adjustment data generated by the position relationship automatically, without relying on manual experience to adjust the pose of the navigation device, which not only improves the accuracy of adjusting the pose of the navigation device, but also improves the efficiency of adjusting the pose of the navigation device.

In 1010, the position relationship between the at least one of the one or more reference markers of the surgical robot and the target field of view of the navigation device is obtained. In some embodiments, operation 1010 may be performed by a pose determination module.

The at least one of the one or more reference markers of the surgical robot refers to one or more markers of the surgical robot that are disposed on components of the surgical robot other than the navigation device (e.g., the end of the mechanical arm, the site to be operated of the target object, etc.). The one or more markers may be in any form. For example, the marker may be a metal reflective ball or a metal reflective sheet, a surface of the metal reflective ball, or the metal reflective sheet is coated with a reflective coating. The surgical robot (which may further be referred to as a system for navigation and positioning of the surgical robot) is an auxiliary system during a surgical procedure that detects and tracks the target object in real time during the surgical procedure, thereby assisting the doctor in performing the surgical operation. The system for navigation and positioning of the surgical robot may include the one or more reference markers and an optical navigation device, and when the target object in real time is detected and tracked by using the optical navigation device, the one or more reference markers usually need to be disposed on the target object, wherein the one or more reference markers is one or more optical markers that are easily recognized. For example, the one or more reference markers may be one or more optical blobs that reflect near-infrared light. By tracking the one or more reference markers using the optical navigation device, real-time pose information of the target object may be obtained.

The one or more reference markers need to be located within the target field of view of the optical navigation device based on the optical characteristics of the optical navigation device. After the target field of view of the optical navigation device is determined, to make the one or more reference markers be located within the target field of view of the optical navigation device, a position relationship between the at least one of the one or more reference markers and the target field of view of the optical navigation device usually needs to be obtained first. The position relationship may be obtained directly based on the position information of the one or more reference markers and the target field of view or may be obtained in other manners after processing the position information of the one or more reference markers and the target field of view.

The one or more reference markers of the surgical robot may include one or more reference markers disposed at the end of the mechanical arm and/or one or more reference markers of the surgical robot disposed on the site to be operated of the target object.

The target field of view refers to a preset size region within the field of view of the navigation device. For example, the target field of view may be a region jointly determined based on a precision range of the mechanical arm, an operation range of the mechanical arm, and a visual field of the navigation device. More detailed descriptions of the target field of view may be found in FIG. 2 and the related description of FIG. 3, which may not be repeated herein.

The position relationship between at least one of the one or more reference markers of the surgical robot and the target field of view of the navigation device may characterize a relative position relationship between the at least one of the one or more reference markers of the surgical robot and the target field of view of the navigation device.

The position relationship between at least one of the one or more reference markers of the surgical robot and the target field of view of the navigation device may be referenced to a relative position and a distance between the at least one of the one or more reference markers of the surgical robot and the target field of view of the optical navigation device, and other information.

For example, the position relationship between the at least one of the one or more reference markers of the surgical robot and the target field of view of the navigation device may include a relative direction between the at least one of the one or more reference markers at the end of the mechanical arm of the surgical robot and the target field of view of the navigation device, an angle between the at least one of the one or more reference markers at the end of the mechanical arm of the surgical robot and the target field of view of the navigation device, a distance between the at least one of the one or more reference markers at the end of the mechanical arm of the surgical robot and the target field of view of the navigation device, or the like. The position relationship between the at least one of the one or more reference markers of the surgical robot and the target field of view of the navigation device may further include a relative direction between the at least one of the one or more reference markers on the site to be operated of the target object and the target field of view of the navigation device, an angle between the at least one of the one or more reference markers on the site to be operated of the target object and the target field of view of the navigation device, and a distance between the at least one of the one or more reference markers on the site to be operated of the target object and the target field of view of the navigation device, or the like.

In some embodiments, the position relationship between the at least one of the one or more reference markers of the surgical robot and the target field of view of the navigation device may include a position relationship between the at least one of the one or more reference markers of the surgical robot and at least one position (e.g., a center point of the target field of view or at least one edge point of the target field of view, etc.) of the target field of view of the navigation device.

In some embodiments, the pose determination module may obtain the position relationship between the at least one of the one or more reference markers of the surgical robot and the target field of view of the navigation device by any process. For example, the position relationship between the at least one of the one or more reference markers of the surgical robot and the target field of view of the navigation device is obtained manually.

In some embodiments, the pose determination module may obtain position information of the at least one of the one or more reference markers of the surgical robot relative to the navigation device, obtain the target field of view of the navigation device, and determine the position relationship between the at least one of the one or more reference markers and the target field of view based on the position information. More descriptions of determining the position relationship between the at least one of the one or more reference markers and the target field of view based on the position information of the at least one of the one or more reference markers of the surgical robot relative to the navigation device may be found in FIG. 11 and the related descriptions, which may not be repeated herein.

In 1020, pose adjustment data is determined based on the position relationship and the pose of the navigation device is adjusted based on the pose adjustment data to make the at least one of the one or more reference markers fall within the target field of view of the navigation device. In some embodiments, operation 1020 may be performed by the pose determination module.

The pose adjustment data refers to data configured to indicate adjusting the pose of the navigation device. For example, the pose adjustment data may include distance adjustment data, posture adjustment data, or the like.

The pose adjustment data may be presented in the form of picture, animation, prompt message, voice, and other forms.

When the pose adjustment data is presented in only one type of the form, for example, when the pose adjustment data is merely displayed in the form of a picture, the pose adjustment information of the navigation device may be adjusted directly based on information on the picture to make the at least one of the one or more reference markers fall within the target field of view of the navigation device. When the pose adjustment data is presented in two or more types of the form, a plurality of types of the pose adjustment data may be configured jointly to adjust the pose of the navigation device, or merely a portion of types of the pose adjustment data may be selected to be configured to adjust the pose of the navigation device. For example, in response to that the pose adjustment data is displayed both in the form of picture and prompting information, the information on the picture and the prompting information may be combined to jointly adjust the pose of the optical navigation device, or merely the information on the picture or the prompting information may be configured to adjust the pose of the navigation device.

In some embodiments, by obtaining a position relationship between at least one of one or more reference markers of the system for navigation and positioning of the surgical robot and a target field of view of the navigation device of the surgical robot, the pose adjustment data is generated based on the position relationship, and the pose of the navigation device adjustment data is generated based on the pose adjustment data, and the pose of the navigation device is adjusted based on the pose adjustment data to make the one or more reference markers to fall within the target field of view of the navigation device. The pose of the optical navigation device is adjusted directly and automatically through the pose adjustment data generated by the position relationship according to the above processes, without relying on manual experience to adjust the pose of the navigation device, not only improves the accuracy of adjusting the pose of the optical navigation device, but also improves efficiency of adjusting the pose of the optical navigation device.

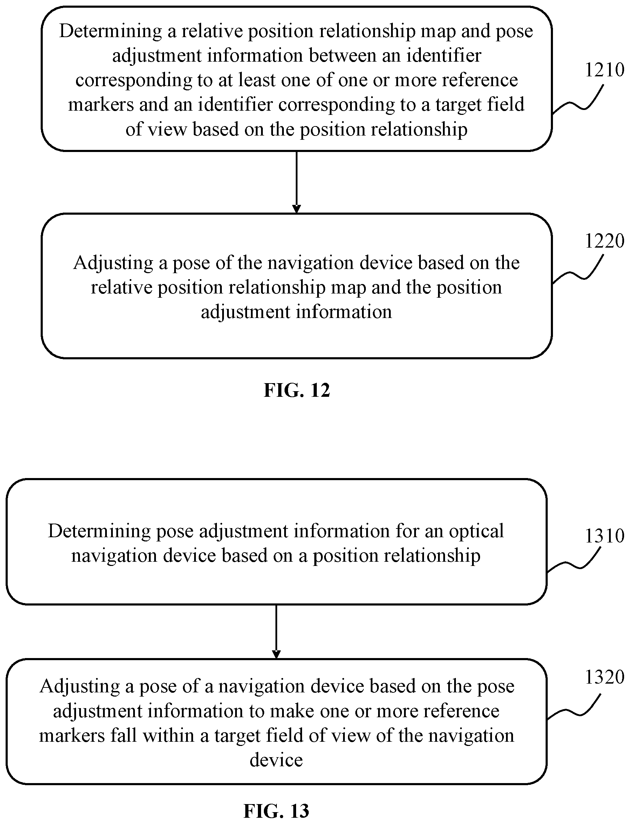

In some embodiments, the pose determination module may determine a relative position relationship map between an identifier corresponding to the at least one of the one or more reference markers and an identifier corresponding to the target field of view and pose adjustment information, and adjust the pose of the navigation device according to the relative position relationship map and the pose adjustment information. More descriptions of adjusting the pose of the navigation device according to the relative position relationship map and the position adjustment information may be found in FIG. 12 and the related descriptions, which may not be repeated herein.

In some embodiments, the position determination module may determine the position adjustment information of the optical navigation device based on the position relationship, and adjust the pose of the optical navigation device based on the position adjustment information to make the at least one of the one or more reference markers fall within the target field of view of the optical navigation device. More descriptions of adjusting the pose of the optical navigation device based on the pose adjustment information may be found in FIG. 13 and the related descriptions, which may not be repeated herein.

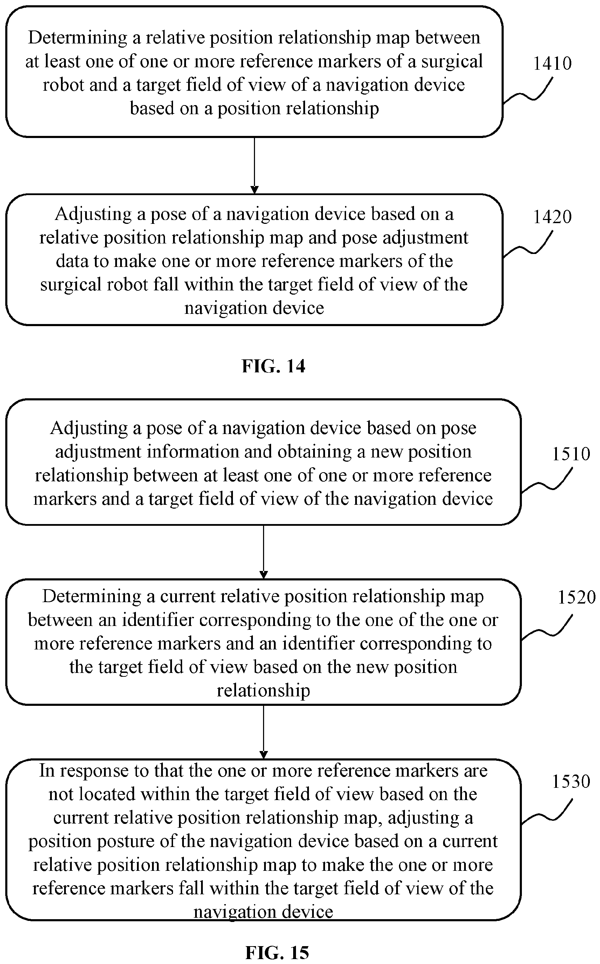

In some embodiments, the pose determination module may determine, based on the position relationship, a relative position relationship map between the at least one of the one or more reference markers of the surgical robot and the target field of view of the navigation device, adjust the target pose of the navigation device based on the relative position relationship map and the pose adjustment data, and cause the one or more reference markers of the surgical robot to be located within the target field of view of the navigation device. More descriptions of adjusting the pose of the navigation device based on the relative position relationship map and the pose adjustment data may be found in FIG. 14 and the related descriptions, which may not be repeated herein.

In some embodiments, the pose determination module may adjust the pose of the navigation device based on the pose adjustment data to make a first reference marker fall within a first visual field region, and adjust the pose of the end effector of the mechanical arm (e.g., a second reference marker) based on the target pose to make the second reference marker disposed at the end of the mechanical arm fall within a second visual field region. The first reference marker and the second reference marker may be located in different portions of the surgical robot, respectively. The first visual field region may be a target field of view or a region within the target field of view, and the second visual field region may be located within the first visual field region.

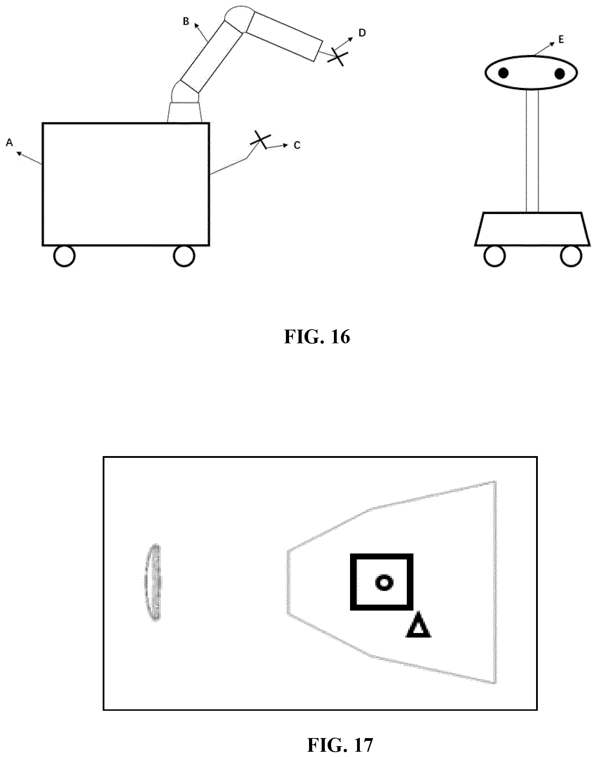

The first reference marker may usually be fixed to a system component that maintains a relative stationary position relationship with the surgical trolley. In some embodiments, the first reference marker may be disposed on a target object fixed on a head fixing device connected with a support arm of the surgical trolley or the surgical trolley. For example, the first reference marker may be provided on a head of the target object. The second reference marker is be disposed on the end effector of the mechanical arm, and the end effector of the mechanical arm (e.g., the second reference marker) does not move during a subsequent adjustment of the pose of the navigation device. As shown in FIG. 16, A denotes the surgical trolley, B denotes the mechanical arm, C denotes the at least one of the one or more reference markers, D denotes the second reference marker disposed at the end of the mechanical arm, and E denotes the navigation device. As shown in FIG. 17, the first reference marker is be identified with "o" and the second reference marker may be identified with "△".

The target field of view includes a first visual field region and a second visual field region, the first visual field region is disposed inside the second visual field region. The first visual field region is a region jointly determined based on the precision range of the mechanical arm, the operation range of the mechanical arm, and the visual field of the navigation device. The second visual field region is an entire target field of view. As shown in FIG. 17, the second visual field region is identified by a hexagonal shape, and the first visual field region is identified by a rectangular box located inside the hexagon. Since both the first reference marker and the second reference marker are fixedly disposed, the pose of the navigation device may be adjusted based on the pose adjustment data to make the first reference marker fall within the first visual field region and the second reference marker fall within the second visual field region.

When adjusting the pose of the navigation device, the pose of the optical navigation device may first be coarsely adjusted in a manual or motor-driven manner to make the pose of the optical navigation device roughly face two reference markers. When the navigation device captures the two reference markers, i.e., when the two reference markers enter the visual field of the navigation device, the optical navigation device is finely adjusted based on the pose adjustment data in the manual or motor-driven manner to make the identifiers representing the two reference markers in the relative position relationship map fall within the corresponding identifiers representing the target field of view of the navigation device.

In some embodiments, a first reference marker falls within the first visual field region and the second reference marker falls within the second visual field region by adjusting the pose of the navigation device based on the pose adjustment data. The adjustment process of the navigation device is guided based on the pose adjustment data, which facilitates the adjustment of the navigation device to ensure that the first reference marker and the second reference marker fall within the corresponding visual field region quickly and accurately. On this basis, the subsequent registration and alignment process are ensured to be smoothly completed, which can avoid the risk of register alignment failure and return to a previous workflow due to the poor field of view of the optical navigation device, and improve the stability of a system workflow.

In some embodiments, the pose of the navigation device is adjusted based on the pose adjustment data to make the first reference marker fall within the first visual field region, and the pose of the end effector of the mechanical arm is adjusted based on the pose adjustment data to make the second reference marker disposed at the end of the mechanical arm fall within the second visual field region. The first reference marker of the surgical robot is disposed on the surgical trolley of the surgical robot or the site to be operated of the target object. The second reference marker is disposed at the end of the mechanical arm of the surgical robot. The first visual field region is disposed within the second visual field region. More description of adjusting the pose of the navigation device based on the pose adjustment data to make the first reference marker fall within the first visual field region and make the second reference marker disposed at the end of the mechanical arm fall within the second visual field region may be found in FIG. 18 and the related descriptions, which may not be repeated herein.

In some embodiments, by obtaining the position relationship between the at least one of the one or more reference markers of the surgical robot and the target field of view of the navigation device, the target pose of the navigation device may be generated more quickly and accurately according to the position relationship, and then the navigation device is controlled to pose based on the target pose of the navigation device, which may reduce the risk of space registration failure due to a poor visual filed of the navigation device.

FIG. 11 is a flowchart illustrating an exemplary process for determining a position relationship between at least one of one or more reference markers and a target field of view based on position information of the at least one of the one or more reference markers of a surgical robot relative to a navigation device according to some embodiments of the present disclosure. As shown in FIG. 11, the process 1100 includes the following operations. In some embodiments, the process 1100 may be performed by the processing device 110.

In 1110, position information of one or more reference markers of the surgical robot relative to the navigation device is obtained. In some embodiments, operation 1110 may be performed by the pose determination module.

The position information of the one or more reference markers of the surgical robot relative to the navigation device may include position information of the one or more reference markers at the end of the mechanical arm of the surgical robot and/or the one or more reference markers on the site to be operated of the target object relative to the navigation device. For example, the one or more reference markers of the surgical robot may include directions of the one or more reference markers of the surgical robot at the end of the mechanical arm of the surgical robot relative to the navigation device, an angle between the one of the one more reference markers of the surgical robot at the end of the mechanical arm of the surgical robot and the navigation device, and a distance between the one of the one more reference markers of the surgical robot at the end of the mechanical arm of the surgical robot and the navigation device, or the like. The position relationship between the end of the mechanical arm of the surgical robot and the navigation device may further include a direction of the one or more reference markers on the site to be operated of the target object relative to the navigation device, an angle between the site to be operated of the target object and the navigation device, a distance of the site to be operated of the target object and the navigation device, or the like.

In some embodiments, the position relationship between the at least one of the one or more reference markers of the surgical robot and the navigation device may include a position relationship between the at least one of the one or more reference markers of the surgical robot and at least one component of the navigation device (e.g., a camera, a near-infrared light source, etc.).

In some embodiments, the pose determination module may obtain the position information of the at least one of the one or more reference markers of the surgical robot relative to the navigation device through any manners. For example, through a near-infrared light source at a front end of the lens of the near-infrared binocular positioning camera, the navigation device may emit near-infrared light to the at least one of the one or more reference markers of the surgical robot within the target field of view, and the near-infrared light may be reflected to a light-sensitive component of the camera through the at least one of the one or more reference markers, causing the at least one of the one or more reference markers to form a bright point of light in the image, so that coordinate information of the at least one of the one or more reference markers in a coordinate system of the navigation device may be extracted, thereby determining the position information of the at least one of the one or more reference markers of the surgical robot relative to the navigation device. As another example, when the one or more reference markers of the surgical robot is located outside the field of view of the navigation device, the pose determination module may obtain an image of the surgical robot, and determine the position relationship between the at least one of the one or more reference markers of the surgical robot and the navigation device based on the image of the surgical robot.

In 1120, a target field of view of the navigation device is obtained. In some embodiments, operation 1120 may be performed by the range determination module.

In some embodiments, the range determination module may obtain the initial receptive field of the navigation device, obtain a space range constraint, constraint the initial receptive field according to the space range constraint, and obtain a target field of view of the navigation device. More descriptions of constraining the field of the initial receptive field according to the space range constraint to obtain the target field of view of the navigation device may be found in FIG. 3 and FIG. 4 and the related descriptions, which may not be repeated herein.

In 1130, a position relationship between at least one of the one or more reference markers and the target field of view based on the position information is determined. In some embodiments, operation 1130 may be performed by the pose determination module.

The position relationship may characterize information such as a relative position and a distance between the at least one of the one or more reference markers and the target field of view of the navigation device.

In some embodiments, the pose determination module may determine a position relationship between the at least one of the one or more reference markers and the target field of view based on the position information in any manner. For example, since the target field of view of the navigation device may be characterized using corresponding coordinate information, the position relationship between the at least one of the one or more reference markers and the target field of view may be characterized directly by the position information between the one or more reference markers and the coordinate information of the target field of view. The pose determination module may further characterize the position relationship between the at least one of the one or more reference markers and the target field of view based on operation results of mathematical operations performed on the position information of the one or more reference markers and the coordinate information of the target field of view.

In some embodiments, by obtaining the position information of the one or more reference markers of the surgical robot relative to the navigation device first, the target field of view of the navigation device is obtained, and the position relationship between the at least one of the one or more reference markers and the target field of view based on the position information is determined, a process for obtaining the position relationship between the at least one of the one or more reference markers and the target field of view may be simple and easy to operate, and the efficiency may be improved.

FIG. 12 is a flowchart illustrating an exemplary process for generating pose adjustment information based on a relative position relationship map and adjusting a pose of a navigation device based on the pose adjustment information according to some embodiments of the present disclosure. As shown in FIG. 12, the process 1200 includes the following operations. In some embodiments, the process 1200 may be performed by the processing device 110.

In 1210, a relative position relationship map and pose adjustment information between an identifier corresponding to the at least one of one or more reference markers and an identifier corresponding to the target field of view is determined based on the position relationship. In some embodiments, operation 1210 may be performed by the pose determination module.

The relative position relationship map may be an image configured to characterize the position information of the at least one of the one or more reference markers of the surgical robot relative to the navigation device. The relative position relationship map may be a two-dimensional (2D) image or a three-dimensional (3D) image in a format such as Joint Photographic Experts Group (JPEG), Tagged Image File Format (TIFF), Graphics Interchange Format (GIF), or the like.

In some embodiments, the pose adjustment data may be presented in a form of the relative position relationship map, and after a position relationship between the at least one of the one or more reference markers and the target field of view of the optical navigation device is obtained, the relative position relationship map may be used to characterize the position relationship. In some embodiments, different identifiers may be employed in the relative position relationship map to represent the one or more reference markers and the target field of view, respectively, wherein the identifiers may be geometric shapes or other types of identifiers. When the one or more reference markers may be not located within the field of view of the optical navigation device, the relative position relationship map may merely include the identifier corresponding to the target field of view. When the at least one of the one or more reference markers falls within the field of view of the optical navigation device, the relative position relationship map may further be refreshed in real time based on the position relationship between the at least one of the one or more reference markers and the target field of view of the optical navigation device.

In 1220, the pose of the navigation device is adjusted based on the relative position relationship map and the pose adjustment information. In some embodiments, operation 1220 may be performed by the pose determination module.

In some embodiments, since the relative position relationship map is configured to characterize a position relationship between the at least one of the one or more reference markers and the target field of view of the optical navigation device, the pose of the optical navigation device may be adjusted according to the relative position relationship map to make the at least one of the one or more reference markers fall within the target field of view of the optical navigation device. Specifically, whether the at least one of the one or more reference markers falling within the target field of view of the optical navigation device may be determined according to the real-time refreshed relative position relationship map during the process of adjusting the optical navigation device, thereby adjusting the pose of the optical navigation device using a manual or motor-driven process.

In some embodiments, based on the position relationship, a relative position relationship map between the identifier corresponding to the at least one of the one or more reference markers and the identifier corresponding to the target field of view is determined; the target pose of the navigation device is generated based on the relative position relationship map, and since the relative position relationship map can intuitively provide a real-time feedback on the current adjustment of the navigation device, the navigation device may be adjusted more efficiently based on the feedback results, thereby improving the accuracy and efficiency of determining the target pose of the optical navigation device's target pose.

FIG. 13 is a flowchart illustrating an exemplary process for adjusting a pose of an optical navigation device based on pose adjustment information according to some embodiments of the present disclosure. As shown in FIG. 12, the process 1300 includes the following operations. In some embodiments, the process 1300 may be performed by the processing device 110.

In 1310, pose adjustment information of the optical navigation device is determined based on a position relationship. In some embodiments, operation 1310 may be performed by the pose determination module.

The pose adjustment data may further be presented in a form of the pose adjustment information. The pose adjustment information refers to information configured to adjust a current pose of the navigation device. In some embodiments, the pose adjustment information may include an adjusted pose of the navigation device, a relative position relationship between the adjusted pose and the one or more reference markers, adjusting a velocity at which the pose, adjusting a time at which the pose, or the like.

In some embodiments, the pose adjustment information may include direction adjustment information and distance adjustment information. The direction adjustment information may be configured to indicate an adjustment direction to the navigation device, wherein the direction may be an upward, downward, leftward, or rightward direction, or an angle of any size in other directions such as a direction of 45 degrees to the left. The distance adjustment information is configured to indicate a moving distance to the navigation device.

In some embodiments, the pose determination module may determine the pose adjustment information of the navigation device based on the position relationship. For example, the direction adjustment information and the distance adjustment information of the navigation device may be determined based on the position relationship. Specifically, the pose determination module may process a mathematical operation on the position information of the one or more reference markers and the coordinate information of the target field of view based on the position relationship to obtain an angle and a distance between the at least one of the one or more reference markers and the target field of view, and determine, in combination with the position relationship, the direction information corresponding to the angle between the at least one of the one or more reference markers and the target field of view. For example, the angle between the at least one of the one or more reference markers and the target field of view is determined to be 45 degrees, the distance is 10 cm, and the direction information corresponding to the angle between the at least one of the one or more reference markers and the target field of view is determined to be a left upward direction in combination with the position relationship, so that the position adjustment information of the navigation device may be obtained to be moved in a direction of the left upward direction of 45 degrees by 10 cm.

In 1320, the pose of the navigation device is adjusted based on the pose adjustment information to make the at least one of the one or more reference markers fall within the target field of view of the navigation device. In some embodiments, operation 1320 may be performed by the pose determination module.

In some embodiments, the pose determination module may adjust the pose of the navigation device based on the pose adjustment information of the navigation device in any manner to make the one or more reference markers of the surgical robot be located within the target field of view of the navigation device. For example, the pose adjustment data may be displayed to a user (e.g., a doctor, a nurse, etc.) through a picture, an animation, a prompting message, a voice, or the like., and the user adjusts the pose of the navigation device based on the pose adjustment information to make the one or more reference markers of the surgical robot is located within the target field of view of the navigator. As another example, the pose adjustment data generates a control instruction based on the pose adjustment information of the navigation device, send the control instruction to a mechanism for adjusting the pose of the navigation device, and the mechanism may automatically adjust the pose of the navigation device based on the control instruction to make the one or more reference markers of the surgical robot be located within the target field of view of the navigation device.

In some embodiments, the pose adjustment information generated by the position relationship, which does not rely on manual experience, can directly automatically adjust the pose of the navigation device, thereby improving the accuracy of adjusting the pose of the navigation device and the efficiency of adjusting the pose of the navigation device. Moreover, adjusting the pose to make the at least one of the one or more reference markers of the surgical robot be located within the target field of view of the navigation device can ensure that the one or more reference markers of the surgical robot is always located within the target field of view during the subsequent process of performing the space registration, to avoid registration failure. Moreover, determining the direction adjustment information and the distance adjustment information of the navigation device based on the position relationship can more clearly indicate the adjustment operation of the navigation device, improving the accuracy and efficiency of adjusting the pose of the navigation device.

FIG. 14 is a flowchart illustrating an exemplary a process for adjusting a pose of a navigation device based on a relative position relationship map and pose adjustment data according to some embodiments of the present disclosure. As shown in FIG. 14, the process 1400 includes the following operations. In some embodiments, the process 1400 may be performed by the processing device 110.

In 1410, a relative position relationship map between at least one of the one or more reference markers of the surgical robot and a target field of view of the navigation device is determined based on the position relationship. In some embodiments, operation 1410 may be performed by the pose determination module.

The relative position relationship map may be an image configured to characterize position information of the one or more reference markers of the surgical robot relative to the navigation device. More descriptions of determining the relative position relationship map may be found in FIG. 12 and the related descriptions, which may not be repeated herein.

In 1420, the pose of the navigation device is adjusted based on the relative position relationship map and the pose adjustment data to make the at least one of the one or more reference markers of the surgical robot be located within a target field of view of the navigation device. In some embodiments, operation 1420 may be performed by the pose determination module.

When both the relative position relationship map and the pose adjustment information are present, merely one of them may be selected to adjust the pose of the optical navigation device.

In some embodiments, when both the relative position relationship map and the pose adjustment information are present, the pose determination module may further adjust the pose of the optical navigation device based on the relative position relationship map and the pose adjustment information together to make the at least one of the one or more reference markers fall within the target field of view of the optical navigation device. The pose determination module may repeatedly generate the pose adjustment information based on the relative position relationship map, adjust the pose of the navigation device based on the pose adjustment information, and obtain a position relationship between one of the one or more adjusted reference markers of the surgical robot and the target field of view of the navigation device, determine an adjusted relative position relationship map based on the one of the one or more adjusted reference markers of the surgical robot, and repeat the above operations until determining that the one or more reference markers of the surgical robot are located within the target field of view based on the adjusted relative position relationship map. More descriptions of adjusting the pose of the navigation device based on the relative position relationship map and the position adjustment information may be found in FIG. 15 and the related descriptions, which may not be repeated herein.

FIG. 15 is a flowchart illustrating an exemplary process for adjusting a pose of a navigation device based on a relative position relationship map and pose adjustment information according to some embodiments of the present disclosure. As shown in FIG. 15, the process 1500 includes the following operations. In some embodiments, the process 1500 may be performed by the processing device 110.

In 1510, a pose of the navigation device is obtained based on the pose adjustment information and a new position relationship between at least one of the one or more reference markers and a target field of view of the navigation device is obtained. In some embodiments, operation 1510 may be performed by the pose determination module.

In some embodiments, the position determination module may adjust the pose of the navigation device in a manual or motor-driven manner based on specific content of the position adjustment information first, and obtain a new position relationship between the at least one of the one or more reference markers and the target field of view of the navigation device based on the adjusted result. The specific process for obtaining the new position relationship may further be obtained based on new position information between the at least one of the one or more reference markers and the target field of view, or may further be obtained after processing the new position information between the at least one of the one or more reference markers and the target field of view by other manners.

In 1520, a current relative position relationship map between an identifier corresponding to at least one of the one or more reference markers and an identifier corresponding to the target field of view is determined based on the new position relationship. In some embodiments, operation 1520 may be performed by the pose determination module.

In some embodiments, after the new position relationship between the at least one of the one or more reference markers and the target field of view of the navigation device is obtained, the pose determination module may refresh an old relative position relationship map, i.e., update the position information of the identifier corresponding to the at least one of the one or more reference markers in the relative position relationship map to obtain a current relative position relationship map between the identifier corresponding to the at least one of the one or more reference markers and the identifier corresponding to the target field of view.

In 1530, in response to that the one or more reference markers determined based on the current relative relationship map are not located within the target field of view, the position posture of the navigation device may be adjusted based on the current relative position relationship map to make the at least one of the one or more reference markers fall within the target field of view of the navigation device. In some embodiments, operation 1530 may be performed by the pose determination module.

In some embodiments, whether the at least one of the one or more reference markers fall within the target field of view of the navigation device may be determined by combining with the current relative position relationship map, and in response to that the one or more reference markers are not within the target field of view of the navigation device, the pose determination module may continue to adjust the pose of the navigation device by using a manual or motor-driven manner according to the relative position relationship between the at least one of the one or more reference markers and the target field of view in the current relative position relationship map until the at least one of the one or more reference markers fall within the target field of view of the navigation device illustrated in the relative position relationship map.

In some embodiments, the relative position relationship map between the identifier corresponding to the at least one of the one or more reference markers and the identifier corresponding to the target field of view and the pose adjustment information of the navigation device is determined by determining the relative position relationship map based on the position relationship. The pose of the navigation device is adjusted based on the relative position relationship map and the pose adjustment information to make the at least one of the one or more reference markers fall within the target field of view of the navigation device. Because the above process may simultaneously adjust the pose of the navigation device based on the relative position relationship map and the position adjustment information, by cooperation of the two types of the pose adjustment data, the accuracy and efficiency of adjusting the pose of the navigation device can be improved.

FIG. 18 is a flowchart illustrating an exemplary process for adjusting a pose of a navigation device based on pose adjustment data according to some embodiments of the present disclosure. As shown in FIG. 18, the process 1800 includes the following operations. In some embodiments, the process 1800 may be performed by the processing device 110.

In some embodiments, the at least one of the one or more reference markers may be disposed on a surgical trolley or an object to be detected of the system for navigation and positioning of the surgical robot.

In 1810, a pose of the navigation device is adjusted based on the pose adjustment data to make at least one of one or more reference markers fall within the first visual field region. In some embodiments, operation 1810 may be performed by the pose determination module.

In some embodiments, the pose determination module may generate the pose adjustment data based on a position relationship between a first reference marker and a target field of view of the navigation device, and adjust the pose of the navigation device based on the pose adjustment data by a manual or automated manner to make the first reference marker fall within the first visual field region. More descriptions of generating the pose adjustment data based on the position relationship between the first reference marker and the target field of view of the navigation device may be found in FIG. 10 and FIG. 11 and the related descriptions, which may not be repeated herein.

In some embodiments, the pose determination module may generate a relative position relationship map based on the position relationship between the first reference marker and the target field of view of the navigation device, and adjust the pose of the navigation device based on the relative position relationship map to make the first reference marker fall within the first visual field region. More descriptions of generating the relative position relationship map based on the position relationship between the first reference marker and the target field of view of the navigation device may be found in FIG. 12 and the related descriptions, which may not be repeated herein.

In 1820, a pose of an end effector of the mechanical arm is adjusted based on the pose adjustment data to make the at least one of the one or more reference markers be disposed at the end of the mechanical arm fall within a second visual field region. In some embodiments, operation 1820 may be performed by the pose determination module.

The second reference marker is disposed on the end effector of the mechanical arm of the system for navigation and positioning of the surgical robot, and the first visual field region is disposed within the second visual field region.

In some embodiments, the pose determination module may generate the pose adjustment data based on a position relationship between the second reference marker and the target field of view of the navigation device, adjust the pose of the end effector of the mechanical arm by a manual or automated manner based on the pose adjustment data to make the second reference marker fall within the second visual field region. More descriptions of generating the pose adjustment data based on the position relationship between the second reference marker and the target field of view of the navigation device may be found in FIG. 10 and FIG. 11 and the related descriptions, which may not be repeated herein.

In some embodiments, the pose determination module may generate a relative position relationship map based on the position relationship between the second reference marker and the target field of view of the navigation device, and adjust the pose of the end effector of the mechanical arm based on the relative position relationship map to make the second reference marker fall within the second visual field region. More descriptions of generating the relative position relationship map based on the position relationship between the second reference marker and the target field of view of the navigation device may be found in FIG. 12 and the related description, which may not be repeated herein.

In some embodiments, the first reference marker is disposed fixedly and the second reference marker is disposed on the mechanical arm fixedly, and the first reference marker and he second reference marker may move with a movement of the mechanical arm. Specifically, the system for navigation and positioning of the surgical robot includes a surgical trolley and a mechanical arm, the one or more reference markers includes the first reference marker and the second reference marker, and the first reference marker is disposed on the surgical trolley or the object to be detected, and the second reference marker is disposed at the end of the mechanical arm. The target field of view includes a first visual field region and a second visual field region, and the first visual field region is disposed within the second visual field region, and the end effector of the mechanical arm may move during subsequent adjustment of the pose of the optical navigation device. Referring to FIG. 16, a second reference marker D disposed at the end of the mechanical arm may drive the second reference marker to move through the movement of the mechanical arm. Since a first reference marker C is fixed, the pose of the optical navigation device may be adjusted based on the pose adjustment data to make the first reference marker fall within the first visual field region.

In a state after operation 1820 is adjusted, since the end effector of the mechanical arm may move, the pose of the end effector of the mechanical arm may be adjusted based on the pose adjustment data by using a manual or motor-driven manner to make the second reference marker disposed at the end of the mechanical arm fall within the second visual field region, and a specific process for adjusting the optical navigation device and the end of the mechanical arm based on the pose adjustment data may be referred to the above embodiments, which may not be repeated herein.

Specifically, when the pose of the end effector of the mechanical arm is adjusted based on the pose adjustment data, since the pose adjustment data refers to information for adjusting the pose of the optical navigation device, for example, the pose adjustment data includes rotating the optical navigation device downwardly, an approximate direction of the field of view of the optical navigation device is determined at this time. Moreover, since the second reference marker needs to fall within the second visual field region, and the second visual field region refers to an entire field of view of the optical navigation device, such that after the approximate direction of the field of view of the optical navigation device is determined, it has a certain guiding role for adjusting the pose of the end effector of the mechanical arm to make the second reference marker fall within the second visual field region of the optical navigation device.

In some embodiments, the pose of the optical navigation device is adjusted by adjusting the pose of the optical navigation device based on the pose adjustment data to make the at least one of the one or more reference markers fall within the first visual field region. The pose of the end effector of the mechanical arm of the mechanical arm is adjusted based on the pose adjustment data to make the second reference marker disposed at the end of the mechanical arm fall within the second visual field region. Since the second reference marker is fixedly disposed on the mechanical arm and is able to move with the movement of the mechanical arm, the second reference marker guides a process of adjusting the pose of the end effector of the mechanical arm based on the pose adjustment data to ensure that the second reference marker falls within the corresponding visual field region quickly and accurately. Moreover, the adjustment process provided in this embodiment adjusts the one or more reference markers to the corresponding visual field region when the one or more reference markers are fixedly disposed on the mechanical arm and moves along with the movement of the mechanical arm, making the use scenarios more flexible and diversified. On this basis, a smooth completion of the subsequent registration and alignment process is ensured, a risk of registration alignment failure caused by the poor field of view of the optical navigation device is avoided and a risk of returning to a previous workflow is avoided, resulting in improving the stability of the system workflow.

In some embodiments, the surgical robot may further include other reference markers of the surgical robot, such as a third reference marker set in a sterilization region, or the like. After a pose of the navigation device is adjusted, the pose determination module may further adjust a pose of a component where the one of the other reference markers is located to make the one of the other reference markers be located in the corresponding visual field region. The visual field region corresponding to the other reference markers may be located inside the second visual field region. The processor adjusting the pose of the component where the one of the other reference markers is located is similar to a processor for adjusting the pose of the end effector of the mechanical arm, more descriptions of adjusting the pose of the component where the one of the other reference markers is located may be found in FIG. 18 and the related descriptions, which may not be repeated herein.

In some embodiments, the relative position relationship map and the pose adjustment information may further be displayed on a display interface. In some embodiments, the relative position relationship map is displayed in a first display region and the pose adjustment information is displayed in a second display region.

The display interface may include a first display region and a second display region. The display interface may be deployed on a system component such as a display, a display panel, or the like, a display for deploying the display interface is a device with a fixed setting, and a display panel for deploying the display interface may be a device that may move accordingly to the movement of the user. The display interface may further be deployed on an optical navigation device or other components in the system for navigation and positioning of the surgical robot, which is not specifically limited by this embodiment. Specifically, as shown in FIG. 19, a first display region 1910 may display a relative position relationship map, and the relative position relationship map may be displayed in a three-view diagram or a three-dimensional diagram. A second display region 1920 may display the pose adjustment information.

In some embodiments, continue to refer to FIG. 19, the first display region 1910 may be presented in a three-view diagram, with a left side being a top view of the relative position relationship map, wherein a half-ellipse on the left side denotes the optical navigation device, a hexagon denotes the second visual field region, a rectangular box inside the hexagon denotes the first visual field region, a first reference marker is identified with "o" and a second reference marker is identified with "△".

The middle is a front view of the relative position relationship map, wherein a shape composed of a plurality of small polygons represents the second visual field region, a rectangular box in the middle of the second visual field region represents a first visual field region, and the first reference marker and the second reference marker are represented in the same manner as the first reference marker and the second reference marker in the top view.

The right is a side view of the relative position relationship map, wherein a small circle on the left side of the diagram represents the optical navigation device, a large trapezoid formed by two small trapezoids on the right side in the view represents a second visual field region, a rectangular box in the middle side in the view of the second visual field region represents a first visual field region, and the first reference marker and the second reference marker are represented in the same manner as the first reference marker and the second reference marker in the top view.

The pose adjustment information may be displayed in the second display region corresponding to a dotted box through text, animation, or the like., continue to refer to FIG 19. Taking the text as an example, in response to that "Optical Tracking System (OTS)" is displayed, an adjustment direction of the optical navigator refers to a downward adjustment. The text may further include distance adjustment information, such as "Turn OTS 10cm downwards", which indicates that the adjustment direction of the optical navigator is vertical adjustment and the adjustment distance is 10cm.

The display interface may further include a third display region. As shown in FIG. 19, the third display region is specifically configured to display a raw image acquired by the optical navigation device, which may typically be image information such as grayscale maps, color maps, or the like.

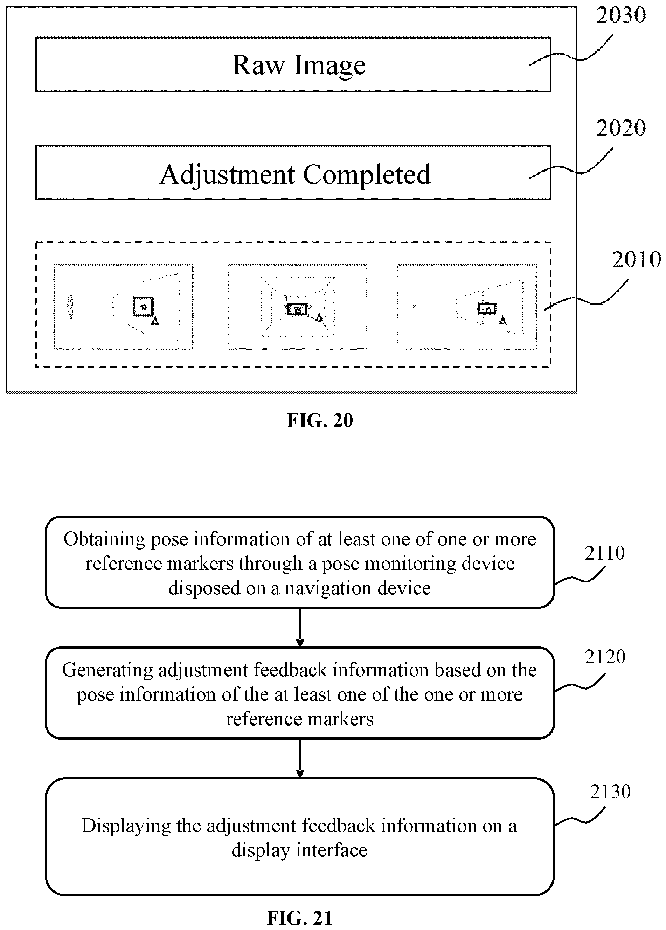

After the adjustment of the pose of the optical navigation device is completed, the final content displayed on the display interface may be as shown in FIG. 20. A third display region 2030 may still display the real-time image acquired by the optical navigation device, a second display region 2020 may display "adjustment is completed" in text form, and the first display region 2010 may display the relative position relationship map after the adjustment is completed.

In some embodiments, the relative position relationship map and the pose adjustment information determined based on the position relationship may be displayed in different display regions, providing an interactive display interface for the user. The user may further operate on the display interface to control the adjustment of the pose of the optical navigation device by a motor drive. Moreover, the real-time feedback on a current adjustment situation may optimize user operation experience and enhance operability.

In some embodiments, after the pose adjustment information is generated, not only can the pose adjustment information be instructed to be displayed on the display interface, but also the pose adjustment information may be output by using voice broadcasting and/or indicator lights. In some embodiments, a voice device such as a microphone may be used to perform a voice broadcast on the pose adjustment information. An LED indicator may further be used to indicate the pose adjustment information. For example, at least one LED indicator may be provided in different directions of the optical navigation device, and the LED indicators in the corresponding directions may be controlled to flash according to an adjustment direction in the pose adjustment information, and a corresponding count of LED indicators may further be controlled to flash according to the adjustment distance in the pose adjustment information.

In some embodiments, a flexible diversity of outputting the pose adjustment information is improved by adopting a voice announcement and/or an indicator light to output the pose adjustment information, thereby ensuring the reliability of outputting the pose adjustment information.

In some embodiments, the process for determining the space registration pose of the surgical robot may further include generating and displaying adjustment feedback information based on the pose information of the navigation device. FIG. 21 is a flowchart illustrating an exemplary process for generating and displaying adjustment feedback information based on the pose information of a navigation device according to some embodiments of the present disclosure. As shown in FIG. 21, operation 2100 includes the following operations. In some embodiments, operation 2100 may be performed by the processing device 110.

In 2110, the pose information of at least one of the one or more reference markers is obtained through a pose monitoring device disposed on the navigation device.

In some embodiments, the pose monitoring device refers to an inertial measurement unit (IMU), a gyroscope, and other positioning and navigation systems deployed on the navigation device. The pose monitoring device may obtain information such as a moving velocity, a yaw angle, and a position of the one or more reference markers through the IMU, and information such as an angle, an angular velocity, an angular acceleration, or the like of the one or more reference markers may be obtained through the gyroscope, and the above information is also known as the pose information of the one or more reference markers.

In 2120, adjustment feedback information is generated based on the pose information of the at least one of the one or more reference markers.

In some embodiments, the adjustment feedback information includes moving distance information of the one or more reference markers and moving velocity information of the one or more reference markers.

The moving distance information of the one or more reference markers and the moving velocity information of the one or more reference markers may be extracted from the pose information of the one or more reference markers to generate the adjustment feedback information. The adjustment feedback information is configured to provide real-time feedback and intelligent evaluation of the adjustment effect of the navigation device.

In 2130, the adjustment feedback information is displayed on the display interface.

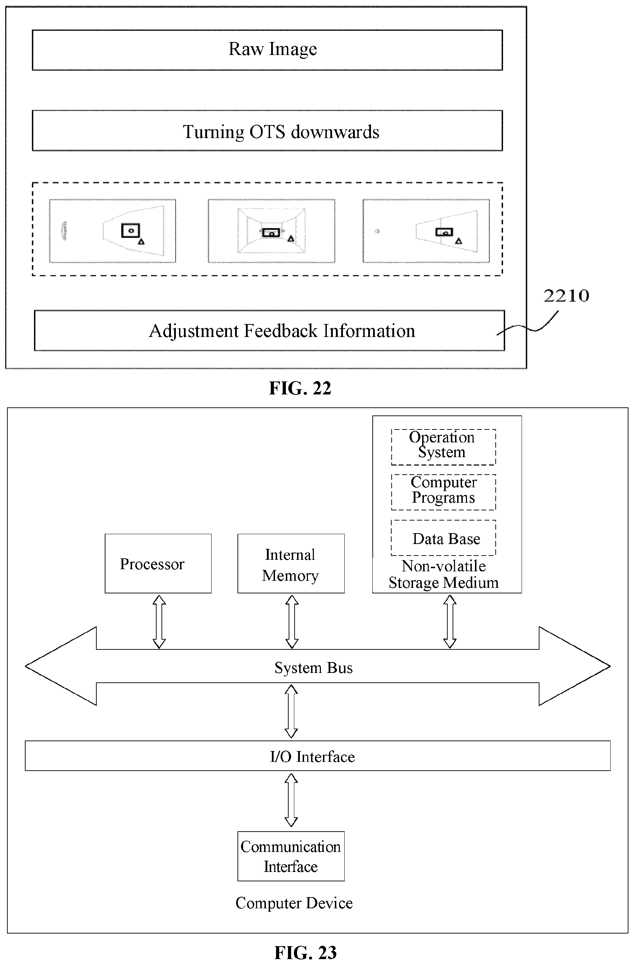

In some embodiments, the adjustment feedback information may further be displayed on the display interface in a form of text, animation, or the like, thereby guiding the user to make further judgments on the current adjustment effect. In some embodiments, as shown in FIG. 22, the display interface may further include a fourth display region 2210, and the fourth display region 2210 is specifically configured to display the adjustment feedback information.

In some embodiments, the pose information of the one or more reference markers is obtained by the pose monitoring device provided on the navigation device. The adjustment feedback information is generated based on the pose information of the one or more reference markers. The adjustment feedback information includes the moving distance information of the one or more reference markers and the moving velocity information of the one or more reference markers. The adjustment feedback information is displayed on the display interface to make further judgments on a current adjustment effect based on the adjustment feedback information, thereby optimizing the user operation experience.

The process for adjusting the pose of the optical navigation device provided in the present disclosure may be applied to a computer device, and the computer device may be a server or a terminal. The server may be a server or a server cluster including a plurality of servers, which is not specifically limited by embodiments of the present disclosure, and the terminal may include but is not limited to a variety of personal computers, laptop computers, smartphones, tablet computers, and portable wearable devices.

Taking the computer device as a server as an example, FIG. 23 is a block diagram illustrating a server according to some embodiments of the present disclosure. As shown in FIG. 23, the computer device includes a processor, a memory, and a network interface connected through a system bus. The processor of the computer device is configured to provide computing and control capabilities. The memory of the computer device includes a non-volatile storage medium and an internal memory. The non-volatile storage medium stores an operating system, computer programs, and a database. An internal memory provides an environment for the operation of an operating system and the computer programs in the non-volatile storage medium. The database of the computer device is configured to store the pose adjustment data for the optical navigation device. A network interface of the computer device is configured to communicate with an external terminal through a network connection. The computer programs are executed by a processor to implement a process for adjusting a pose of an optical navigation device.

It should be understood by those skilled in the art that the structure illustrated in FIG. 23, which is merely a block diagram illustrating a portion of the structures related to the present disclosure embodiment, does not constitute a limitation on the server on which the present disclosure embodiment is applied, and optionally the server may include more or fewer components than those shown in the drawings, or a combination of some of the components, or have different arrangements of components.

In some embodiments, in an orthopedic surgical navigation system, after the process for adjusting the pose of an optical navigation device is completed by using the process provided in each of the above embodiments, in subsequent registration alignment session, continue to refer to FIG. 16, a first reference marker C may be used as a reference array, and a second reference marker D may be used as a registration array. Further, the system needs to complete a registration alignment of a mechanical arm B with an optical navigation coordinate system with the help of the optical navigation device E and a registration array. To avoid an impact on the registration alignment accuracy caused by the unintended jitter of the optical navigation device during a registration alignment process, the jitter may be compensated by disposing the reference array fixed on the surgical trolley A.

Specifically, when the jitter of the optical navigation device E is compensated by the reference array, position information of the registration array obtained is accurate if the optical navigation device E does not undergo the jitter, and when the optical navigation device E undergoes the jitter, the position information of the registration array obtained is deviated. However, since a reference array is fixed to the surgical trolley A, a relative position relationship between the reference array and the registration array is fixed regardless of whether or not jitter occurs in the optical navigation device E. Therefore, true position information of the registration array may be determined based on the relative position relationship when the jitter occurs in the optical navigation device E, thereby realizing compensation for the unintended jitter occurring in the optical navigation device E.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended to those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this specification are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations thereof, are not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software-only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various embodiments. This method of disclosure, however, is not to be interpreted as reflecting an intention that the claimed subject matter requires more features than are expressly recited in each claim. Rather, claimed subject matter may lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the count of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Therefore, by way of example, but not of limitation, alternative configurations of the embodiments of the application may be utilized in accordance with the teachings herein. Accordingly, embodiments of the present application are not limited to that precisely as shown and described.

## Claims

1. A method for determining a space registration pose of a surgical robot, comprising:
determining a target field of view of a navigation device;
adjusting a pose of the navigation device based on the target field of view;
determining the space registration pose of the surgical robot according to the target field of view and a pose of an end effector of a mechanical arm of the surgical robot.

2. The method of claim 1, wherein the determining the target field of view of the navigation device includes:
obtaining an initial receptive field of the navigation device;
obtaining a space range constraint; and
obtaining the target field of view of the navigation device by constraining the initial receptive field according to the space range constraint.

3. The method of claim 2, wherein the space range constraint includes at least one of a constraint of the mechanical arm, a constraint of a target object, and a constraint of a field of view of the navigation device.

4. The method of claim 2, wherein the obtaining the space range constraint, and obtaining the target field of view of the navigation device by constraining the initial receptive field according to the space range constraint includes:
constructing an objective function for describing a space range of the target field of view;
performing a constraint calculation on the objective function according to the space range constraint through an optimization algorithm; and
determining the target field of view based on a constrained objective function.

5. The method of claim 1, wherein the adjusting the pose of the navigation device based on the target field of view includes:
after determining the target field of view of the navigation device, adjusting a positioning of the navigation device according to the target field of view to make the end effector of the mechanical arm of the surgical robot be located within the target field of view.

6. The method of claim 1, wherein the determining the space registration pose of the surgical robot based on the target field of view and the pose of the end effector of the mechanical arm of the surgical robot includes:
obtaining a plurality of position coordinates and a plurality of rotation postures of the end effector of the mechanical arm; and
determining the space registration pose of the surgical robot based on the plurality of position coordinates and the plurality of rotation postures.

7. The method of claim 6, wherein obtaining the plurality of rotation postures of the end effector of the mechanical arm includes:
obtaining a plurality of initial rotation postures of the end effector of the mechanical arm; and
obtaining a plurality of rotation postures by deflecting the plurality of initial rotation postures to a same direction.

8. The method of claim 7, wherein the obtaining the plurality of initial rotation postures of the end effector of the mechanical arm includes:
obtaining a maximum posture range of the end effector of the mechanical arm; and
determining the plurality of initial rotation postures of the end effector of the mechanical arm in the maximum posture range according to a count of postures and/or a posture dispersion of the end effector of the mechanical arm.

9. The method of claim 1, wherein the adjusting a pose of the navigation device based on the target field of view includes:
obtaining a position relationship between at least one of one or more reference markers of the surgical robot and the target field of view of the navigation device; and
generating pose adjustment data based on the position relationship and adjusting the pose of the navigation device based on the pose adjustment data to make the at least one of the one or more reference markers fall within the target field of view of the navigation device.

10. The method of claim 9, wherein the generating pose adjustment data based on the position relationship and adjusting the pose of the navigation device based on the pose adjustment data includes:
determining a relative position relationship map and pose adjustment information between an identifier corresponding to at least one of the one or more reference markers and an identifier corresponding to the target field of view based on the position relationship; and
adjusting the pose of the navigation device according to the relative position relationship map and the pose adjustment information.

11. The method of claim 10, wherein the adjusting the pose of the navigation device according to the relative position relationship map and the pose adjustment information includes:
adjusting the pose of the navigation device based on the pose adjustment information and obtaining a new position relationship between the at least one of the one or more reference markers and the target field of view of the navigation device;
determining a current relative position relationship map between the identifier corresponding to the at least one of the one or more reference markers and the identifier corresponding to the target field of view based on the new position relationship; and
in response to determining that the at least one of the one or more reference markers is not located within the target field of view determined based on the current relative position relationship map, adjusting the pose of the navigation device based on the current relative position relationship map to make the at least one of the one or more reference markers fall within the target field of view of the navigation device.

12. The method of claim 9, wherein the one or more reference markers includes a first reference marker and a second reference marker; and
the generating pose adjustment data based on the position relationship and adjusting the pose of the navigation device based on the pose adjustment data includes:
adjusting the pose of the navigation device based on the pose adjustment data to make the first reference marker fall within a first visual field region and make the second reference marker fall within a second visual field region, wherein the first reference marker is disposed on a surgical trolley of the surgical robot or a target object, the second reference marker is disposed at an end of the mechanical arm, the second visual field region is the target field of view, and the first visual field region is located within the second visual field region.

13. The method of claim 9, wherein the one or more reference markers includes a first reference marker and a second reference marker; and
the generating pose adjustment data based on the position relationship and adjusting the pose of the navigation device based on the pose adjustment data includes:
adjusting the pose of the navigation device based on the pose adjustment data to make the first reference marker fall within a first visual field region; and
adjusting a pose of the end effector of the mechanical arm based on the pose adjustment data to make the second reference marker disposed at an end of the mechanical arm fall within a second visual field region;
wherein the first reference marker is disposed on a surgical trolley of the surgical robot or a target object, the second visual field region is the target field of view, and the first visual field region is located within the second visual field region.

14. The method of claim 9, further comprising:
obtaining pose information of the navigation device through a pose monitoring device;
generating adjustment feedback information based on the pose information of the navigation device, wherein the adjustment feedback information includes moving distance information of the navigation device and moving velocity information of the navigation device; and
displaying the adjustment feedback information on a display interface.

15. The method of claim 9, wherein the obtaining a position relationship between the one of one or more reference marker of the surgical robot and the target field of view of the navigation device includes:
obtaining position information of the at least one of the one or more reference markers of the surgical robot relative to the navigation device;
obtaining the target field of view of the navigation device; and
determining a position relationship between the at least one of the one or more reference markers and the target field of view based on the position information.

16. A system for determining a space registration pose of a surgical robot, comprising:
a range determination module configured to determine a target field of view of a navigation device;
a pose determination module configured to adjust a pose of the navigation device based on the target field of view; and
a pose registration module configured to determine a space registration pose of the surgical robot according to the target field of view, a target pose of the navigation device, and an initial pose of an end effector of a mechanical arm of the surgical robot.

17. A method for obtaining a visual navigation device receptive field, comprising:
obtaining an initial receptive field of a navigation device in a surgical robot;
obtaining a space range constraint; and
obtaining a target field of view of the navigation device by constraining the initial receptive field according to the space range constraint.

18. A method for adjusting a pose of an optical navigation device, comprising:
obtaining a position relationship between a reference marker of a surgical robot and a target field of view of a navigation device of the surgical robot;
generating pose adjustment data based on the position relationship; and
adjusting a pose of the navigation device based on the pose adjustment data to make the reference marker fall within the target field of view of the navigation device.

19. A device for determining a space registration pose of a surgical robot, comprising a processor, wherein the processor is configured to perform the method for determining the space registration pose of the surgical robot of any one of claims 1 to 15, the method for obtaining the visual navigation device receptive field of claim 17, or the method for adjusting the pose of the optical navigation device of claim 18.

20. A non-transitory computer-readable storage medium, the storage medium storing computer instructions, and when the computer reads the computer instructions in the storage medium, the computer executes the method for determining the space registration pose of the surgical robot of any one of claims 1 to 15, the method for obtaining the visual navigation device receptive field of claim 17, or the method for adjusting the pose of the optical navigation device of claim 18.

21. A surgical robot, comprising a navigation device, a mechanical arm, and a processor, wherein
the processor is configured to obtain an initial receptive field of the navigation device; and
the processor is configured to obtain a space range constraint and obtain a target field of view of the navigation device by constraining the initial receptive field according to the space range constraint.

22. A system for adjusting a pose of a surgical robot, including one or more reference markers, an optical navigation device, and a computer device, and the optical navigation device communicating with the computer device, wherein:
the optical navigation device is configured to acquire position information of the reference marker and send the position information to the computer device; and
the computer device is configured to determine a position relationship between at least one of the one or more reference markers and a target field of view of the optical navigation device; generate pose adjustment data based on the position relationship, and adjust a pose of the optical navigation device based on the pose adjustment data to make the at least one of the one or more reference markers fall within the target field of view of the navigation device.

23. The system of claim 22, wherein the one or more reference markers includes a first reference marker and a second reference marker, the first reference marker is disposed on a surgical trolley of the surgical robot or a target object of a system for navigation and positioning of the surgical robot, and the second reference marker is disposed on an end of a mechanical arm of the system for navigation and positioning of the surgical robot.

24. The system of claim 22, further comprising a display, wherein
the display is configured to display the pose adjustment data;
the pose adjustment data includes at least one of a relative position relationship map and pose adjustment information, and the pose adjustment data is determined based on the position relationship.
